# EUROPEAN PATENT SPECIFICATION

(11) **EP 3 162 330 B1**
(45) Date of publication and mention of the grant of the patent: **05.09.2018**
(21) Application number: 16195424.3
(22) Date of filing: 25.10.2016
(51) Int. Cl.: A61F 13/02, A61F 13/06

(54) **DRESSING AND DRESSING ASSEMBLY FOR PREVENTING PRESSURE ULCERS**
VERBAND UND VERBANDANORDNUNG ZUR VERMEIDUNG VON DRUCKGESCHWÜREN
PANSEMENT ET ENSEMBLE PANSEMENT POUR LA PRÉVENTION DES PLAIES DE PRESSION

(30) Priority: 27.10.2015 US 201562246750 P; 18.12.2015 US 201562269193 P
(43) Date of publication of application: 03.05.2017
(73) Proprietor: Hill-Rom Services, Inc., Batesville, IN 47006-9167 (US)
(72) Inventor: RIBBLE, David L, Batesville, IN Indiana 47006-9167 (US); EMMONS, Kirsten M, Batesville, IN Indiana 47006-9167 (US); AGDEPPA, Eric D, Batesville, IN Indiana 47006-9167 (US); LACHENBRUCH, Charles A, Batesville, IN Indiana 47006-9167 (US); BEDEL, David L, Batesville, IN Indiana 47006-9167 (US)
(74) Representative: Findlay, Alice Rosemary

(56) References cited:
- WO-A1-2010/080907
- US-A- 5 704 905
- US-A1- 2013 030 343

## Description

The subject matter described herein relates to a dressing and a dressing assembly for preventing pressure ulcers throughout a portion the human posterior region extending longitudinally from approximately the sacrum to the ischeal tuberosities and laterally across the gluteal muscles.

Individuals such as hospital patients who are immobilized in a bed for an extended time are susceptible to developing pressure ulcers. One anatomical region of special concern is the patient's posterior, particularly the region extending longitudinally from approximately the sacrum to the ischeal tuberosities (ITs) and laterally across the gluteal muscles particularly the gluteus maximus and gluteus medius. Pressure ulcers in the vicinity of the sacrum and the gluteal muscles are often the result of moisture on the skin and friction and shear acting on or experienced by the skin or the underlying tissue. Pressure ulcers associated with the ischeal tuberosities are sometimes a form of pressure ulcer referred to as a deep tissue injury (DTI). In their early stages of development deep tissue injuries might not present any external signs of their existence. As a result the presence of a deep tissue injury is often difficult to detect until the injury breaks through the skin, by which time the condition is painful, susceptible to infection, and difficult to treat successfully. Accordingly, it is desirable to develop ways of preventing the development of pressure ulcers. It is particularly desirable to develop devices that prevent pressure ulcers across a large portion of the posterior even though the preventive measures preferred for one anatomical sub-region (e.g. the sacral region) may differ from the preventive measures preferred for another anatomical sub-region (e.g. the ITs).

In one aspect the invention provides a dressing for guarding against the development of pressure ulcers which has a recipient side and an environmental side. The dressing comprises a moisture barrier layer whose components include a sacral member, a left gluteal member and a right gluteal member. Each gluteal member has a base and an extension which extends longitudinally inferiorly further than the inferior edge of the sacral member by a dimension which is approximately no less than the longitudinal length of the sacral member. The dressing also includes a connective element on the recipient side of the dressing. The connective element is arranged to provide a zone of occlusion. In some embodiments the dressing includes electrodes and is a component of a dressing assembly which includes an exciter for stimulating gluteal muscles.

The invention will now be further described by way of example with reference to the accompanying drawings, in which:
**FIG. 1** is a plan view of a dressing as seen from the environmental side thereof with dashed lines segmenting the dressing into members useful for describing certain embodiments.
**FIG. 2** is an elevation view in the direction **2--2** of FIG. **1** showing a moisture barrier layer and a connective element and also showing the dressing applied to the skin of a care recipient.
**FIG. 3A** is a view similar to that of FIG. **1** showing the dressing applied to a care recipient.
**FIGS. 3B** and **3C** are views showing human anatomical features useful for describing the dressing in relation to human anatomy.
**FIG. 4** is a plan view showing a variant of the dressing in which inner edges of the gluteal extensions converge more decidedly toward each other than do the inner edges of the variant of FIG. **1****.**
**FIGS. 5-12** are a series of plan views of a dressing as seen from the recipient side thereof and a set of elevation views showing various arrangements of the connective element of the dressing.
**FIGS. 13-14** are a plan view and an elevation view showing a zone of occlusion for a dressing in which the connective element is an adhesive layer which is spatially coextensive with or nearly spatially coextensive with the moisture barrier layer.
**FIGS. 15-16** are a plan view and an elevation view similar to those of FIGS. **13-14** showing a zone of occlusion for a dressing in which the connective element is a strip of adhesive.
**FIG. 17** is an elevation view illustrating a connective element embodied as an adhesive layer having one-way liquid absorbancy.
**FIG. 18** is an elevation view illustrating a connective element embodied as an adhesive layer having one-way liquid permeability.
**FIGS. 19-30** are a series of plan views of a dressing as seen from the recipient side thereof and a set of elevation views showing various arrangements of the connective element of the dressing with the connective element broken away to expose an auxiliary layer embodied as a moisture management layer, specifically a liquid absorbant layer.
**FIGS. 31-42** are a series of plan views of a dressing as seen from the recipient side thereof and a set of elevation views showing various arrangements of the connective element of the dressing with the connective element broken away to expose an auxiliary layer embodied as a moisture management layer, specifically a liquid permeable layer.
**FIGS. 43-50** are a series of plan views of a dressing as seen from the recipient side thereof and a set of elevation views showing various arrangements of the connective element of the dressing with the connective element broken away to expose an auxiliary layer embodied as a pressure equalizing layer.
**FIGS. 51A-51C** are elevation views illustrating the functioning of the pressure equalizing layer.
**FIG. 51D** is an elevation view showing a dressing with a pressure equalization zone whose thickness exceeds the baseline thickness of the dressing outside the planform of the pressure equalization zone and in which the local increase in thickness is attributable to the thickness of the auxiliary layer.
**FIG. 51E** is an elevation view showing a dressing with a pressure equalization zone whose thickness exceeds the baseline thickness of the dressing outside the planform of the pressure equalization zone and in which the local increase in thickness is attributable to a local increase in thickness of moisture barrier layer.
**FIG. 51F** is an elevation view of a dressing with a pressure equalizing zone having a customizable thickness attributable to multiple peel-away sublayers.
**FIG. 51G** is an illustration of a stockpile of classified dressings in which the thickness of the pressure equalizing zone in a given class differs from the thickness of zone **P_{ZE}** in the other classes.
**FIGS. 52-59** are a series of plan views of a dressing as seen from the recipient side thereof and a set of elevation views showing various arrangements of the connective element of the dressing and also showing an auxiliary layer embodied as a pressure equalizing layer in conjunction with a moisture management layer.
**FIGS. 60-64** are views illustrating example variations on the planform of the dressing.
**FIG. 65** is a view similar to those of FIGS. **60-64** showing a dressing in which a sacral member, a left gluteal member, and a right gluteal member are individual members rather than integrated into a single unit.
**FIGS. 66-67** are a side elevation view and a posterior view illustrating threat-susceptible regions of a target anatomy in connection with a description of criteria for sizing and proportioning the dressing in relation to features of human anatomy.
**FIGS. 68-69** are views similar to the view of FIG. **3C** showing criteria for sizing and proportioning the dressing in relation to features of human anatomy.
**FIG. 70** shows a plan view of a preventive dressing assembly which comprises a dressing having an array of four electrodes and in which the dressing assembly also comprises an exciter for exciting the electrodes.
**FIG. 71** is a chart showing example excitation patterns for the electrodes of the dressing assembly of FIG. **70** and also enumerating the gluteal muscle or muscles stimulated by each sample excitation pattern.
**FIG. 72** shows a plan view of a preventive dressing assembly which comprises a dressing having an array of six electrodes and in which the dressing assembly also comprises an exciter for exciting the electrodes.
**FIG. 73** is a chart showing example excitation patterns for the electrodes of the dressing assembly of FIG. **72** and also enumerating the gluteal muscle or muscles stimulated by each sample excitation pattern.
**FIGS. 74-75** are a plan view and an elevation view of a dressing having a nonocclusive external layer and a nonocclusive connective element embodied as a distributed adhesive, and showing fluid ingress through the external layer into a zone of coverage and fluid ingress into the connective element and into the zone of coverage.
**FIGS. 76-77** are a plan view and an elevation view of an embodiment similar to that of FIGS. **74-75** but in which the connective element is embodied as an adhesive strip.
**FIGS. 78-79** are a plan view and an elevation view of an embodiment similar to that of FIGS. **76-77** but in which the connective element **120** is embodied as an adhesive strip **190** which blocks moisture transfer laterally and longitudinally into zone **Z_{c}.**

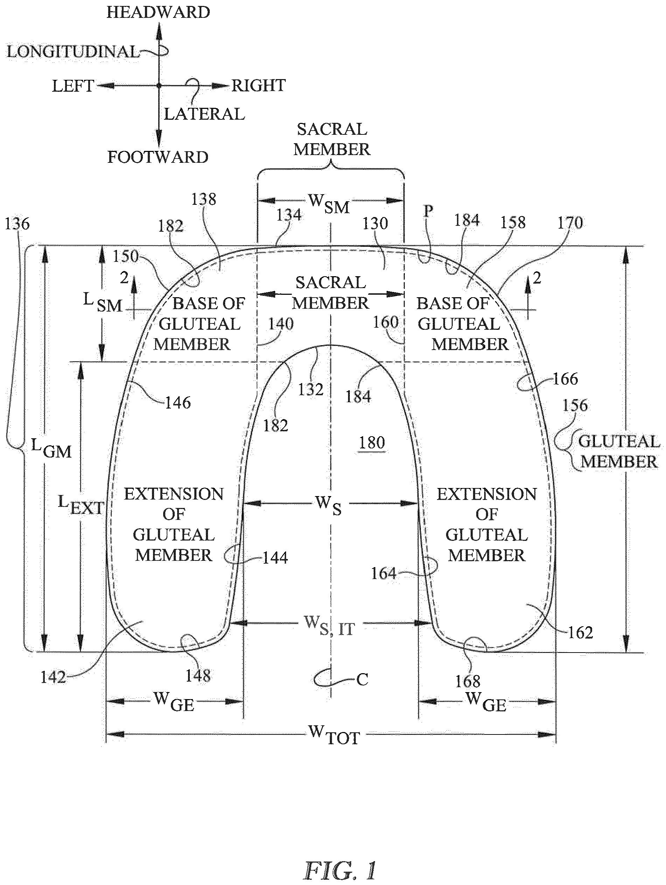

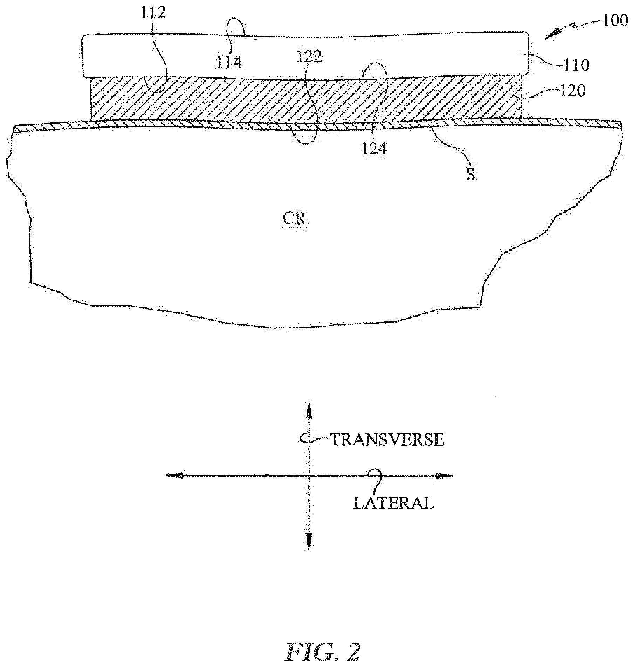

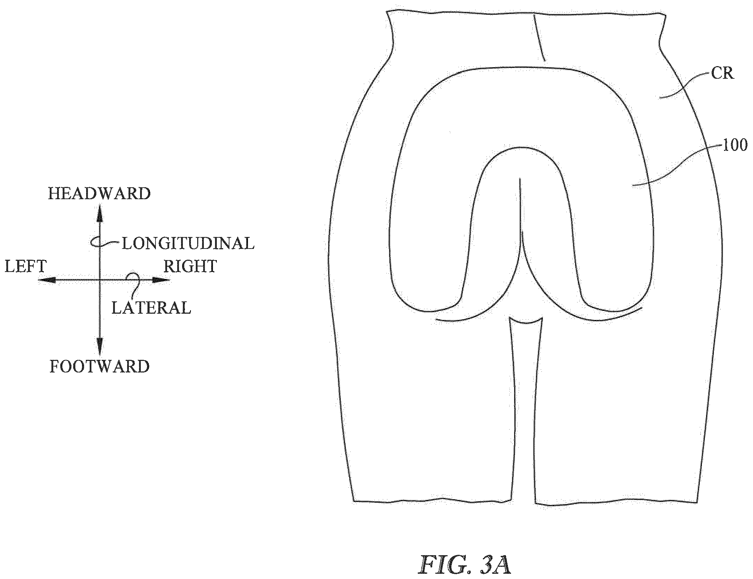

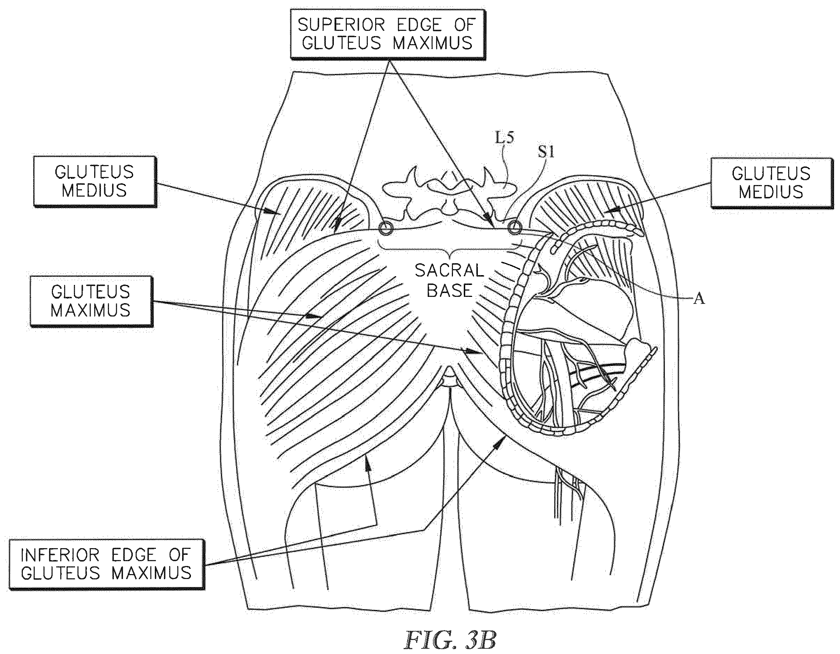

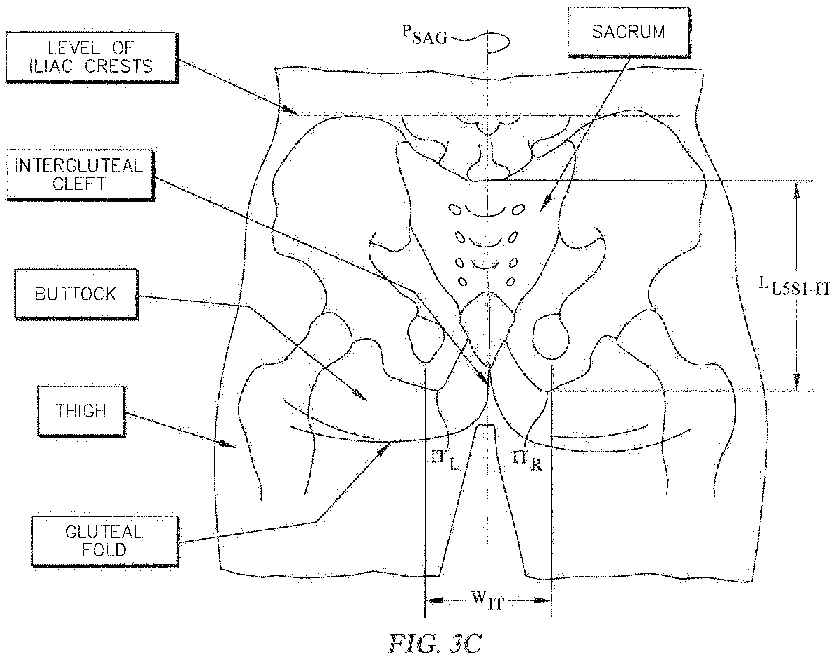

FIGS. **1-2** are schematic illustrations of an example preventive dressing **100** for a care recipient **CR** such as a hospital patient. FIG. **3A** shows the dressing applied to the care recipient. FIGS. **3B** and **3C** show anatomical features useful for describing the dressing in relation to human anatomy and for understanding its operation. Selected illustrations also include reference axes indicating longitudinal, lateral, and transverse directions. When describing the dressing, the thickness of the dressing or its components is the dimension in the transverse direction. Throughout this description features similar to or the same as features previously described may be identified by the same reference numerals. Features which are specific examples of more general features may also all be identified by the same or similar reference numerals.

The dressing is configured to cover a portion of a human body, specifically a posterior region extending longitudinally from approximately the sacrum to the ischeal tuberosities (ITs) and laterally across the gluteal muscles. The dressing is in the form of a thin sheet having sufficient flexibility that it can conform to anatomical contours. In the interest of clarity the thickness of the dressing may be exaggerated in the accompanying illustrations.

The dressing comprises a moisture barrier layer **110** having a recipient side **112** intended to face toward the care recipient and an environmental side **114** which is the side intended to face away from the care recipient. The environmental side is exposed to the external environment. The dressing also has a connective element **120** on the recipient side of the moisture barrier layer. The connective element has a recipient side **122** facing toward the care recipient and an environmental side **124** facing away from the care recipient but not necessarily exposed to the environment. The connective element is intended to hold the dressing in contact with the care recipient and is shown with its recipient side **122** in contact with the care recipient's skin **S.** One example of a connective element is an adhesive which adheres well to human skin but can be removed from the skin without undue difficulty or undue discomfort to the care recipient.

The dressing also has a perimeter **P** and a longitudinally extending centerline **C.** As seen in the plan view of FIG. **1** the dressing has a sacral member **130** corresponding approximately to the sacrum of a care recipient having a target anatomy, for example the anatomy of a 50th percentile United States male. The sacral member has a longitudinal length **L_{SM}**, a lateral width **W_{SM}**, an inferior edge **132** and a superior edge **134.** Superior edge **134** of the sacral member may also be considered to be the superior edge of the dressing as a whole. Width **W_{SM}** is approximately the width of the sacral base of the target care recipient. Accordingly, sacral member **130** extends laterally leftwardly to no more than about the location **"A"** (FIG. **3B**) of the superior edge of the gluteus maximus muscle. The dressing also includes a left gluteal member **136** having a base **138** whose right edge **140** borders the left side of the sacral member, and a left extension **142** having a laterally inner edge **144,** a laterally outer edge **146** and a terminus or inferior edge **148.** Collectively, inner edge **144** outer edge **146** and terminus or inferior edge **148** define a margin of the left extension. The left extension extends longitudinally inferiorly of the inferior edge **132** of the sacral member by extension dimension **L_{EXT}. L_{EXT}** is approximately no less than the longitudinal length **L_{SM}** of the sacral member. The dressing also includes a right gluteal member **156** having a base **158** whose left edge **160** borders the right side of the sacral member, and a right extension **162** having a laterally inner edge **164,** a laterally outer edge **166,** and a terminus or inferior edge **168.** Collectively, inner edge **164,** outer edge **166** and terminus or inferior edge **168** define a margin of the right extension. Taken together extension inferior edges **148, 168** define an inferior edge of the dressing as a whole. The right extension extends longitudinally inferiorly of the inferior edge **132** of the sacral member by the dimension **L_{EXT}**. The overall longitudinal length of the gluteal members, and therefore of the dressing as a whole, is **L_{GM}** which is approximately equal to the sum of **L_{SM}** plus **L_{EXT}.**

The gluteal extensions each have a width **W_{GE}**. The extensions define an intermember space **180** having a width **W_{S}** measured about midway between the inferior edges **148, 168** of the extensions and the inferior edge **132** of the sacral member. The dressing has a total width of **W_{TOT}** which is **W_{S}** plus twice **W_{GE}**.

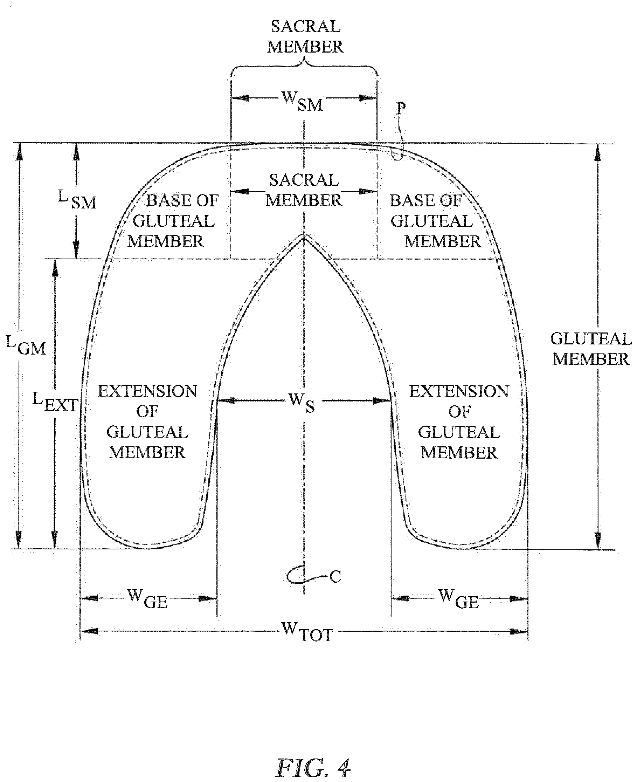

In the dressing of FIG. **1** inner edges **144, 164** of the gluteal extensions and the inferior edge **132** of the sacral member are approximately linear. Curved edge segments **182, 184** connect inferior edge **132** of the sacral member to the inner edges **144, 164** of the extensions. In another embodiment shown in FIG. **4****,** the inner edges converge more decidedly toward each other.

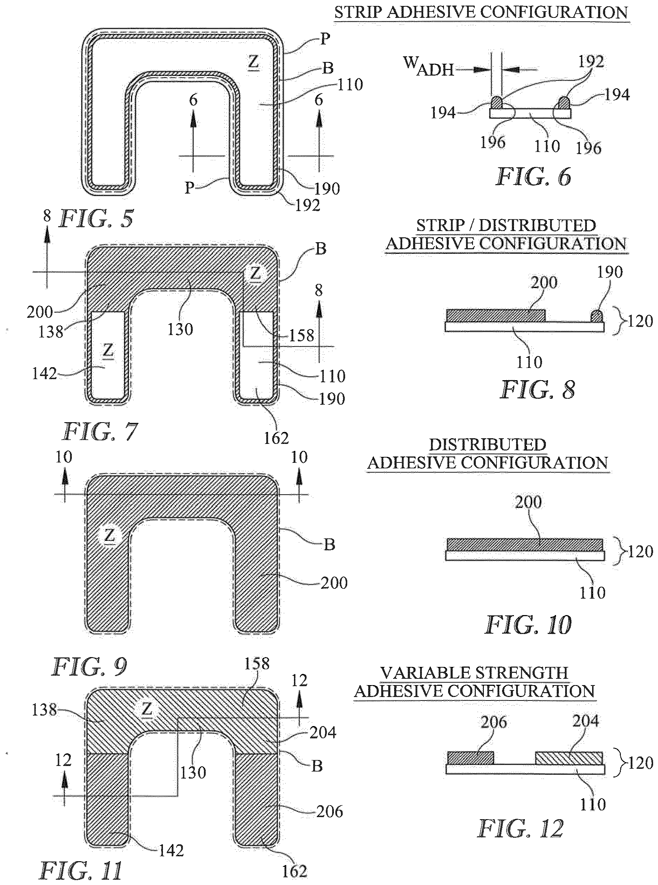

FIGS. **5-12** show various arrangements of connective element **120.** In FIGS. **5-6** the connective element is a continuous strip of adhesive **190** extending along substantially the perimeter of the dressing, i.e. at perimeter **P** or near the perimeter but slightly offset therefrom to define a nonadhesive border **192** of the moisture barrier layer. The adhesive strip has an outer edge **194,** which is the edge closer to dressing perimeter **P,** and an inner edge **196,** which is the edge further away from the dressing perimeter. The configuration of FIGS. **5-6** is referred to as the strip adhesive configuration. In FIGS. **7-8** the connective element comprises a sacral adhesive layer **200** distributed over substantially the entirety of the recipient side of the sacral member **130** and over substantially the entirety of the gluteal bases **138, 158.** The connective element of FIGS. **7-8** further comprises a continuous strip of adhesive **190** extending along substantially the margins of the gluteal member extensions **142, 162.** The arrangement of FIGS. **7-8** is referred to as the strip/distributed adhesive configuration. In FIGS. **9-10** the connective element comprises an adhesive **200** which forms substantially all of the recipient side of the dressing. In other words the recipient side of the dressing is the recipient side of the adhesive. The arrangement of FIGS. **9-10** is referred to as the distributed adhesive configuration.

As seen in FIGS. **11-12****,** the strength of the adhesive need not be spatially uniform as indicated by the density of the crosshatching. In FIGS. **11-12** the connective element comprises a first adhesive composition **204** forming substantially all of the recipient sides of sacral member **130** and gluteal bases **138, 158,** and a second adhesive composition **206** forming substantially all of the recipient side of gluteal extensions **142, 162.** The first adhesive composition adheres relatively more strongly to human skin and the second adhesive composition adheres relatively more weakly to human skin. The configuration of FIGS. **11-12** is referred to as the variable strength adhesive configuration.

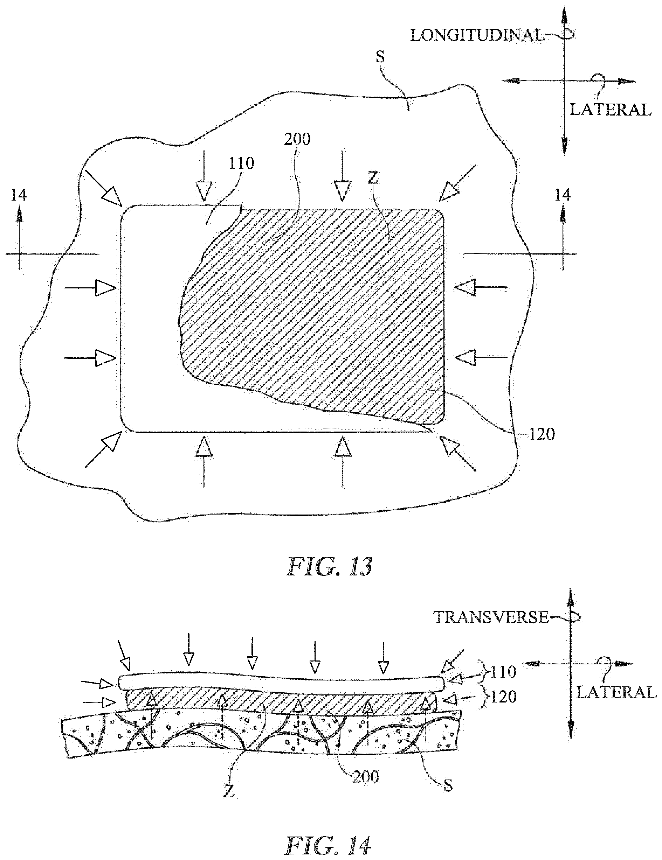

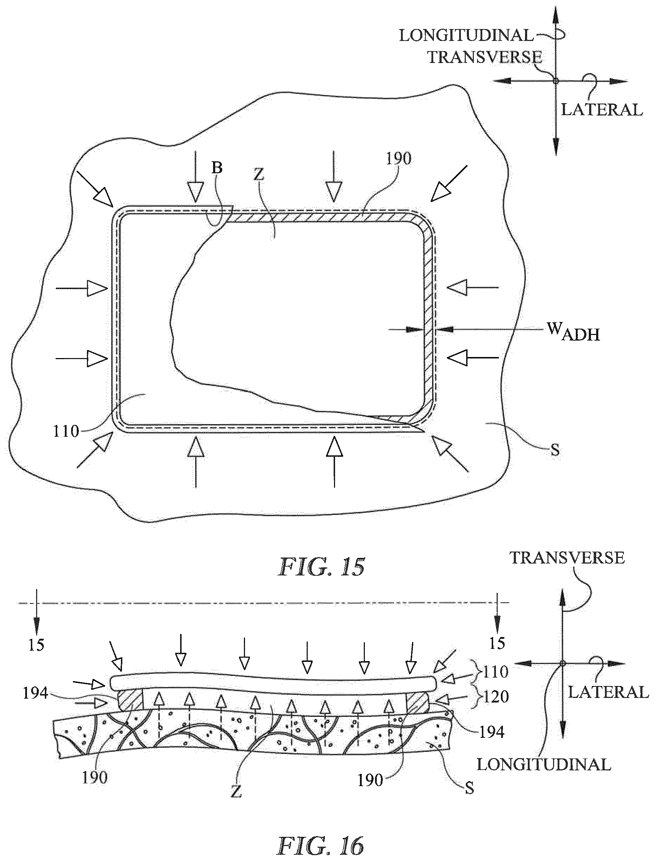

The adhesive or other connective element is arranged to provide a zone of occlusion **Z.** As used herein a zone of occlusion is a zone protected from environmental moisture ingress by the adhesive and the moisture barrier layer. Such protection is desirable because moisture on the skin is a factor that can lead to development of pressure ulcers. Examples of environmental moisture include liquids originating outside the zone of occlusion (e.g. accidentally spilled water, or urine from an incontinent patient). Examples of environmental moisture also include the care recipient's perspiration through skin not covered by the zone of occlusion. The protection against environmental moisture ingress is illustrated schematically in FIGS. **13-14** and **15-16** where the moisture barrier layer **110** protects against environmental moisture ingress principally in the transverse (thickness) direction while the adhesive protects against environmental moisture ingress in the lateral and longitudinal directions (solid arrows which terminate outside the occlusion zone). In the arrangement shown schematically in FIGS. **13-14** the adhesive layer **120** is spatially coextensive with or nearly spatially coextensive with the moisture barrier layer **110.** The zone of occlusion is defined by the spatial extent of the adhesive. In the arrangement shown schematically in FIGS. **15-16** the adhesive layer is a strip of adhesive **190** which forms a closed boundary defined by dashed borderline **B.** (Borderline **B** in FIG. **15** and in other illustrations coincides with outer edge **194** of the adhesive strip but is shown slightly offset edge **194** to preserve the visibility of both the edge and the borderline.) The zone of occlusion is defined by the adhesive strip and includes the region within the closed boundary. In other words the zone of occlusion includes the width **W_{ADH}** of the adhesive strip.

Moisture in the form of the care recipient's perspiration through the skin covered by the zone of occlusion (dashed arrows) of FIGS. **14** and **16** is not environmental moisture. Therefore the status of zone **Z** as a zone of occlusion in FIGS. **13-16** is not defeated by such perspiration.

Returning momentarily to FIGS. **5, 7, 9** and **11****,** dashed borderline **B** shows the zones of occlusion **Z** for each of the described adhesive configurations. In FIG. **5** zone **Z** is the zone enclosed by outer edge **194** of adhesive strip **190** (or equivalently borderline **B**) In FIGS. **7, 9** and **11** zone **Z** is the planform of the entire dressing because the spatially distributed adhesive **200** extends to dressing perimeter **P** (FIGS. **9, 11**) and because outer edge **194** of adhesive strip **190** (FIG. **7**) is exactly at perimeter **P.**

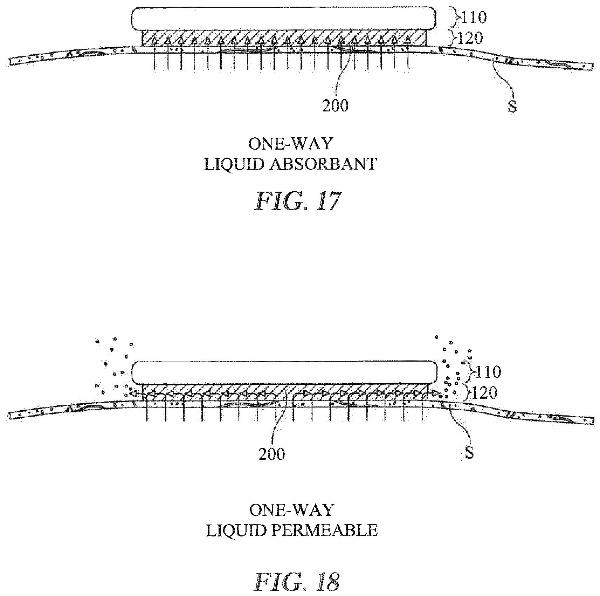

If the adhesive or other connective element is a strip as in FIGS. **5-6****,** or as shown along the margins of the gluteal extensions in FIGS. **7-8****,** the adhesive may or may not be one-way liquid absorbant. However in the examples of this specification the strip adhesive is always considered to be one-way liquid absorbant. If the adhesive is distributed as in the remainder of FIGS. **7-8** or as in FIGS. **9-10****,** the adhesive is one-way liquid absorbant. As shown schematically in FIG. **17** the direction of the one-way absorbancy is the direction that draws moisture, such as perspiration, away from the care recipient's skin **S** thereby eliminating a risk factor (excessive moisture on the skin) for pressure ulcer development. Alternatively, as seen in FIG. **18****,** the adhesive may be one-way liquid permeable. The direction of the one-way permeability is the direction that transfers moisture away from the care recipient's skin. The one-way liquid permeability not only draws moisture away from the care recipient's skin (similar to the liquid absorbant layer of FIG. **17**) but also discharges the moisture to the environment where it can evaporate or be actively disposed of.

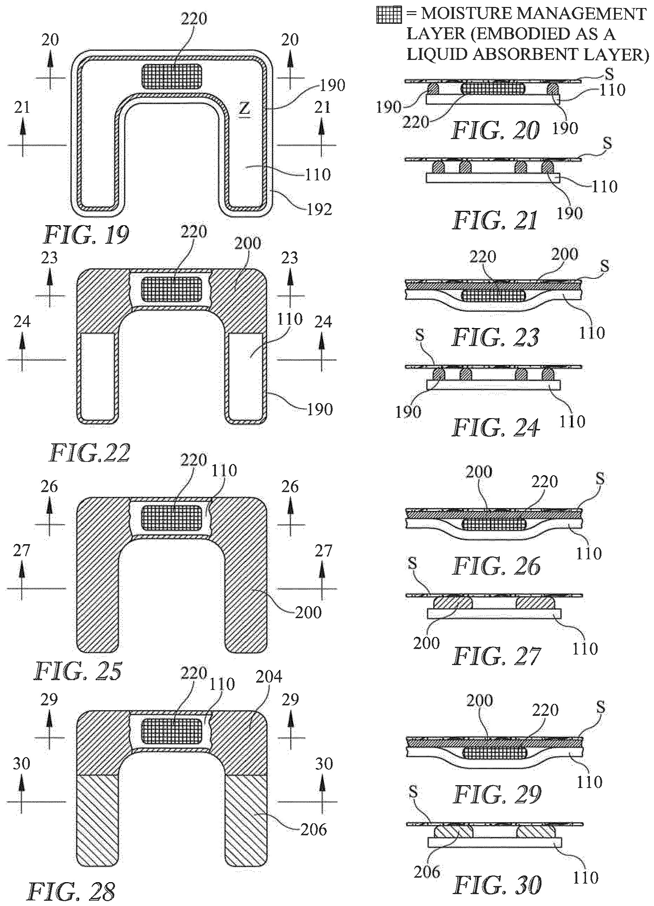

FIGS. **19-50** and **52-59** are similar to FIGS. **5-12** but show an auxiliary layer **220** in addition to the moisture barrier layer **110** and the adhesive layer (embodied as a spatially distributed layer **200** or as an adhesive strip **190**). The auxiliary layer comprises a liquid absorbant layer, a liquid permeable layer, a pressure equalizing layer or some combination of such layers. This specification employs the phrase "moisture management layer" to mean the liquid absorbant layer when used alone, the liquid permeable layer when used alone, or the liquid absorbant layer and liquid permeable layer used together.

FIGS. **19-30** are similar to FIGS. **5-12** but show the auxiliary layer **220,** embodied as a moisture management layer, specifically a one-way liquid absorbant layer. The direction of the one-way absorbancy is the direction that transfers moisture, such as perspiration, away from the care recipient's skin **S,** not toward the care recipient's skin (FIGS. **19-21**) or that transfers moisture away from the adhesive (FIGS. **22-30**) similar to the one-way liquid absorbancy of adhesive **200** of FIG. **17****.** FIGS. **19-****21** show the liquid absorbant layer **220** used in the strip adhesive configuration (e.g. the configuration of FIGS. **5-6**). In FIGS. **19-21** the liquid absorbant moisture management layer resides in the zone of occlusion. When applied to a care recipient the moisture management layer is in direct contact with the care recipient's skin **S.** The illustrated moisture management layer is confined to the sacral member, but could be present elsewhere in the occlusion zone instead of or in addition to being present in the sacral member. In the limit the moisture management layer is present throughout the zone of occlusion but not where it would intervene between adhesive strip **190** and the care recipient's skin. The liquid absorbant moisture management layer draws moisture away from the care recipient's skin and continues to do so until it reaches saturation. FIGS. **22-24** show liquid absorbant layer **220** used in the strip/distributed adhesive configuration (e.g. the configuration of FIGS. **7-8**). FIGS. **25-27** show the liquid absorbant layer **220** used in a distributed adhesive configuration (e.g. the configuration of FIGS. **10-11**). FIGS. **28-30** show the liquid absorbant layer **220** used in the variable strength adhesive configuration (e.g. the configuration of FIGS. **11-12**). In the arrangements of FIGS. **19-30****,** the moisture management **220** layer resides in the zone of occlusion and is transversely between moisture barrier layer **110** and the adhesive or other connective element **120** (which is illustrated as a distributed adhesive **200** in FIGS. **19-30**).

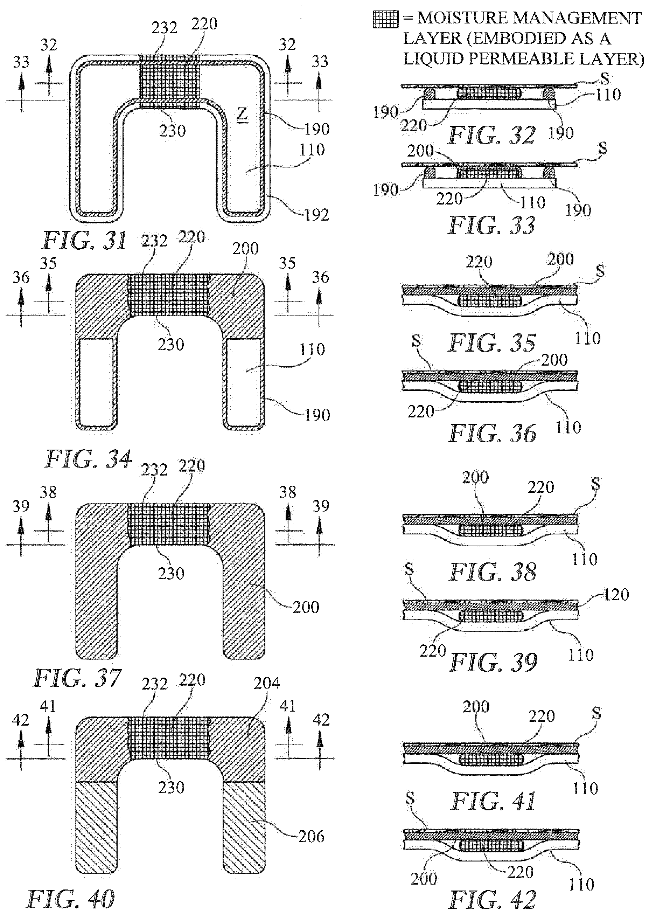

FIGS. **31-42** are similar to FIGS. **5-12** but show the moisture management layer embodied as a one-way liquid permeable layer **220.** The direction of the one-way permeability is the direction that transports moisture, such as perspiration, away from the care recipient's skin **S,** not toward the care recipient's skin (FIGS. **31-33**) or that transports moisture away from the adhesive (FIGS. **34-42**) similar to the one-way liquid permeability of adhesive **200** of FIG. **18****.** A noteworthy difference between the liquid permeable layer of FIGS. **31-42** and the liquid absorbant layer of FIGS. **19-30** is that the liquid permeable layer is in communication with or otherwise exposed to the external environment. In FIGS. **31-42** the liquid permeable layer is exposed to the environment by virtue of extending longitudinally to inferior edge **132** and superior edge **134** of sacral member **130.** Because inferior and superior edges **230, 232** of the liquid permeable layer are exposed to the environment, liquid absorbed by the layer can be discharged to the environment. However because the one-way permeability of the liquid permeable layer is in a direction that transports liquid away from the zone of occlusion, the fact that edges **230, 232** are exposed to the environment does not compromise the occlusive nature of the zone of occlusion.

FIGS. **31-33** show liquid permeable layer **220** used in the strip adhesive configuration (e.g. the configuration of FIGS. **5-6**). In FIGS. **31-33** the liquid permeable moisture management layer resides in the zone of occlusion. When applied to a care recipient the moisture management layer is in direct contact with the care recipient's skin **S.** The illustrated moisture management layer is confined to the sacral member, but could be present elsewhere in the occlusion zone instead of or in addition to being present in the sacral member. In the limit the moisture management layer is present throughout the zone of occlusion but not where it would intervene between adhesive strip **190** and the care recipient's skin. The liquid permeable moisture management layer draws moisture away from the care recipient's skin and discharges it to the environment. FIGS. **34-36** show the moisture permeable layer **220** used in the strip/distributed adhesive configuration (e.g. the configuration of FIGS. **7-8**). FIGS. **37-****39** show the moisture permeable layer **220** used in a distributed adhesive configuration (e.g. the configuration of FIGS. **10-11**). FIGS. **40-42** show the moisture permeable layer **220** used in the variable strength adhesive configuration (e.g. the configuration of FIGS. **11-12**). In the arrangements of FIGS. **31-42****,** the moisture management layer resides in the zone of occlusion and is transversely between moisture barrier layer **110** and the adhesive or other connective element **120** which is illustrated as a distributed adhesive 200 in FIGS. **31-42****.**

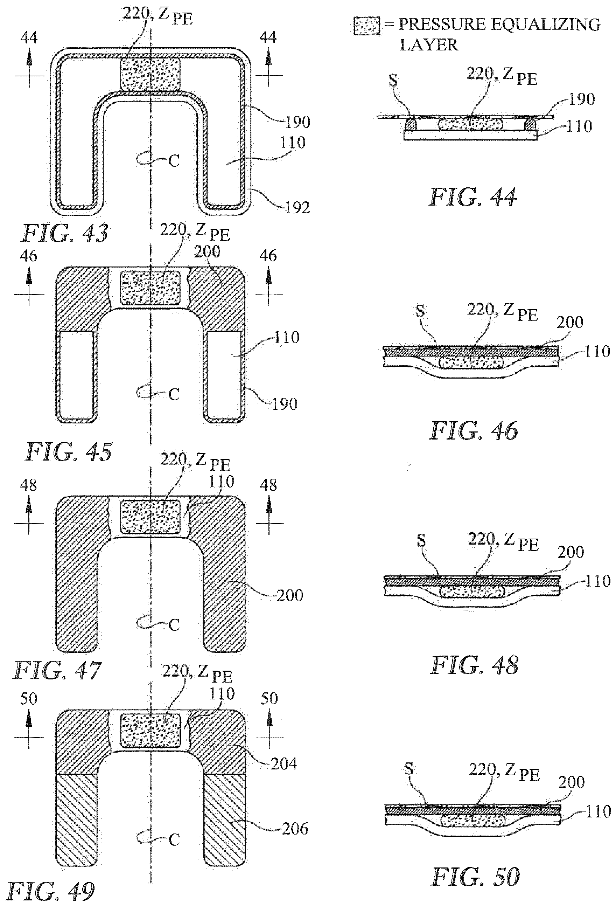

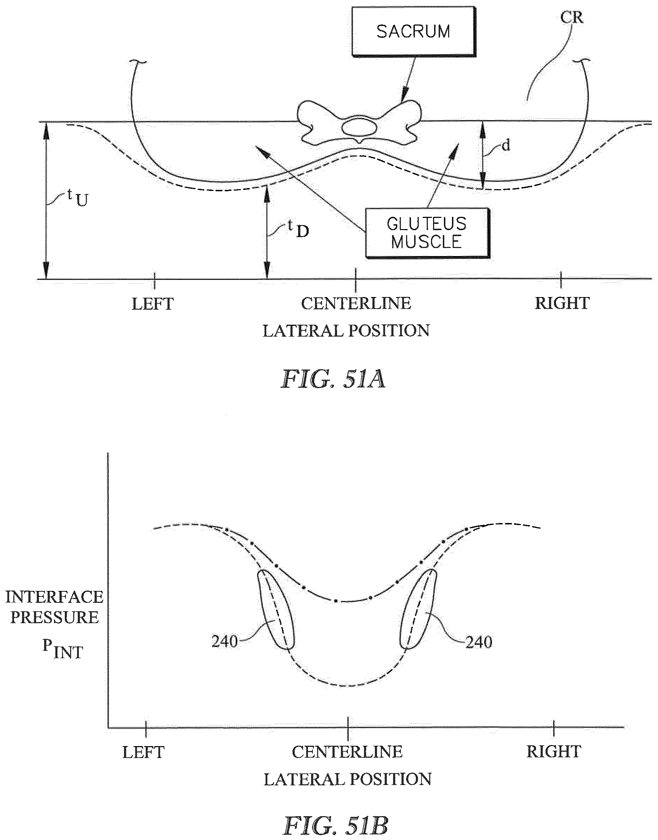

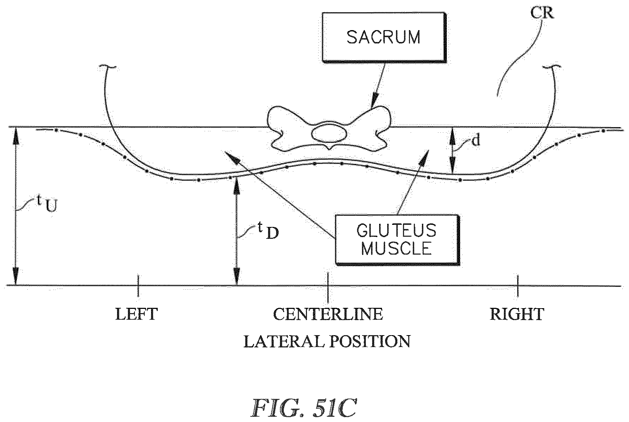

FIGS. **43-50** are similar to FIGS. **5-12** but show the auxiliary layer embodied as a pressure equalizing layer **220.** FIGS. **51A-51C** illustrate the function of the pressure equalizing layer. Referring first to FIG. **51A****,** a mattress has an undeflected thickness **t_{U},** which is substantially uniform in the lateral direction. The weight of the care recipient **CR** deforms the mattress to a laterally nonuniform deflected thickness **t_{D}** as indicated by the dashed line profile. The deflection ***d*** is relatively small in the vicinity of the care recipient's sacrum and larger along the gluteal muscles. Referring additionally to the dashed line of FIG. **51B**, the corresponding interface pressure **P_{INT}** is therefore smaller at the sacrum and larger along the gluteal muscle. The pressure gradients at the steep portions **240** of FIG. **51B** correspond to a region of high shear both at the skin and transversely through the muscle tissue. Referring to FIGS. **43-50** and **51C,** the pressure equalizing layer **220** is a layer of material which spans laterally across centerline **C** of the dressing in the zone of occlusion to define a pressure equalizing zone **Z_{PE}**. The thickness and/or stiffness of the pressure equalizing material layer causes the pressure equalizing zone to exhibit a stiffness (i.e. a resistance to deflection) which is greater than the stiffness of the dressing laterally to the left and right of the pressure equalizing zone. As seen in FIG. **51C** compared to FIG. **51A****,** this causes thickness **t_{D}** and deflection ***d*** to be more laterally uniform. As a result the lateral gradient of interface pressure is reduced (FIG. **51B** dot-dashed profile versus the dashed profile) and therefore so is shear.

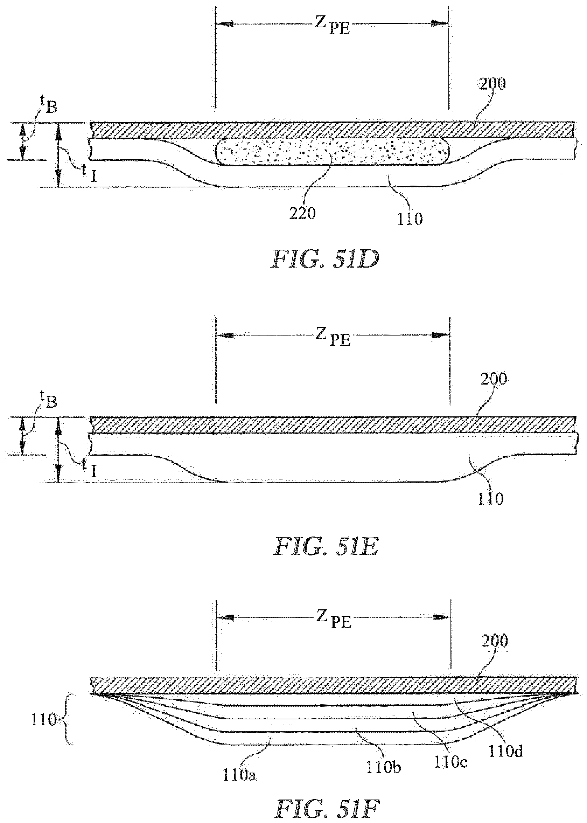

As described above the thickness of the pressure equalizing layer may cause the pressure equalizing zone to exhibit an increased stiffness in comparison to regions of the dressing outside the planform of the pressure equalizing layer and pressure equalizing zone. However an increased thickness of the pressure equalizing layer, relative to the thickness of the dressing outside the pressure equalizing zone/layer, may be sufficient to create a pressure equalizing zone even if the increased thickness is not accompanied by an increase in stiffness. In that case the increased thickness of the pressure equalizing layer acts on its own to yield the pressure equalization of FIG. **51B**, although the increased thickness may diminish patient comfort. FIGS. **51D** and **51E** each show a dressing with a pressure equalization zone whose thickness **t_{I}** exceeds the baseline thickness **t_{B}** of the dressing outside the planform of the pressure equalization zone. In FIG. **51D** the local increase in thickness is attributable to the thickness of auxiliary layer **220.** In FIG. **51E** the local increase in thickness is attributable to a local increase in thickness of moisture barrier layer **110.**

FIG. **51F** shows a dressing with a pressure equalizing zone having a customizable thickness. The customizable thiickness is the result of moisture barrier layer **110** being comprised of multiple peel-away sublayers, for example **110a, 110b, 110c, 110d such** that sublayer **110a** can be peeled off sublayer **110b,** sublayer **110b** can be peeled off sublayer **110c** and sublayer **110c** can be peeled off sublayer **110d.** In practice a caregiver may leave all the sublayers in place or may peel away one or more sublayers. As successive sublayers are removed the pressure equalizing property of the pressure equalizing zone is diminished.

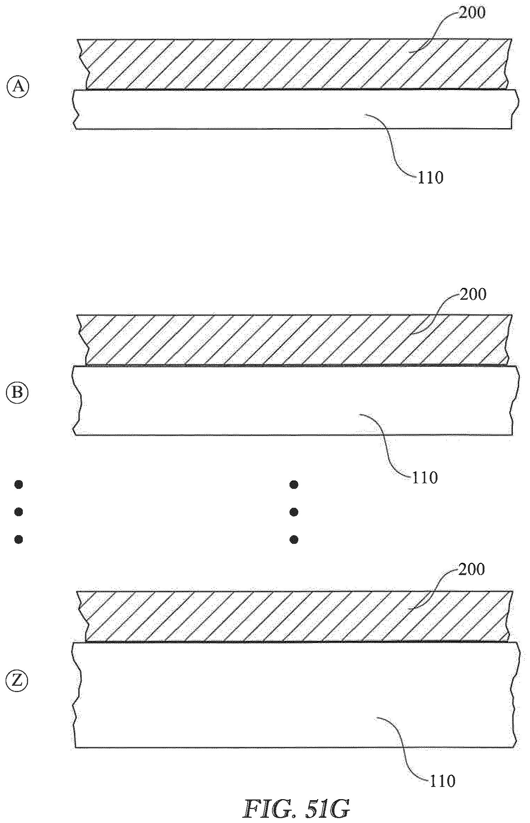

FIG. **51G** shows a variation on the theme of FIG. **51F**. FIG. **51G** shows a stockpile of classified dressings, for example classes A through Z. Zone **P_{ZE}** is a classified zone having a class specific thickness. The thickness of zone **P_{ZE}** in a given class differs from the thickness of zone **P_{ZE}** in the other classes. A manufacturer would produce dressings in each of two or more classes each of which corresponds to a class specific thickness. In practice the caregiver selects a dressing from the class he believes is best suited for the patient under his care.

In one embodiment the pressure equalizing zone is confined to the sacral member, i.e. is confined to the immediate vicinity of the care recipient's sacrum, and does not extend laterally to edges **140, 160** of gluteal bases **138, 158.** In another embodiment the pressure equalizing zone extends laterally to the edges **140, 160** of the gluteal bases. Either way the stiffness of the pressure equalizing zone is greater than the stiffness of the left gluteal base **138** and is also greater than the stiffness of the right gluteal base **158.** In yet another embodiment the pressure equalizing zone may extend beyond edges **140, 160** and into the gluteal bases. In addition, the dash-dot profile of FIG. **51B** demonstrates that the phrase "pressure equalizing" does not mean that the pressure equalizing layer must cause the interface pressure to be substantially uniform in the lateral direction, only that it attenuate the lateral pressure gradients.

FIGS. **43-44** show pressure equalizing layer **220** used in the strip adhesive configuration of the dressing (e.g. the configuration of FIGS. **5-6**). FIGS. **45-46** show the pressure equalizing layer **220** used in the strip/distributed adhesive configuration of the dressing (e.g. the configuration of FIGS. **7-8**). FIGS. **47-48** show the pressure equalizing layer **220** used in the distributed adhesive configuration (e.g. the configuration of FIGS. **10-11**). FIGS. **49-50** show the pressure equalizing layer **220** used in the variable strength adhesive configuration (e.g. the configuration of FIGS. **11-12**). In the arrangements of FIGS. **45-50** the pressure equalizing layer is transversely between moisture barrier layer **110** and the adhesive or other connective element **120.**

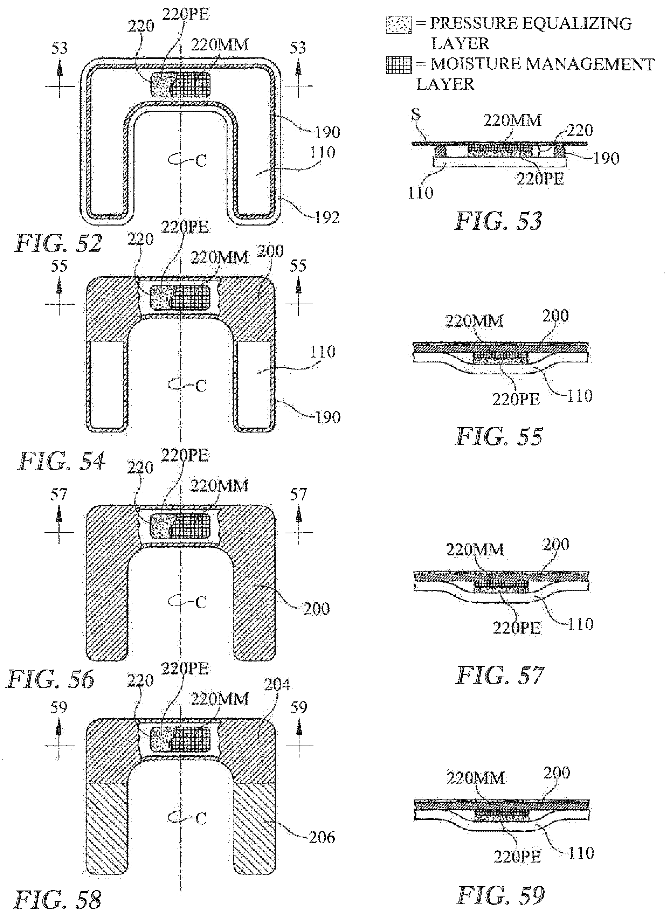

FIGS. **52-59** are similar to FIGS. **5-12** but show the auxiliary layer **220** embodied as a pressure equalizing layer **220PE** in conjunction with a moisture management layer **220MM.** The moisture management layer may be a liquid absorbant layer as previously described, a liquid permeable layer as previously described, or both. As seen in FIGS. **53, 55, 57** and **59** moisture management layer **220MM** is on the recipient side of the pressure equalizing layer so that when the dressing is applied to a care recipient the moisture management layer is in direct contact with the care recipient's skin **S** or is closer to the skin than is the pressure equalizing layer. The pressure equalizing layer **220PE** spans laterally across centerline **C** of the dressing in the zone of occlusion to define a pressure equalizing zone. In one embodiment the pressure equalizing zone is confined to the sacral member, i.e. is confined to the immediate vicinity of the care recipient's sacrum, and does not extend laterally to edges **140, 160** of gluteal bases **138, 158.** In another embodiment the pressure equalizing zone extends laterally to the edges **140, 160** of the gluteal bases. Either way the stiffness of the pressure equalizing zone is greater than the stiffness of the left gluteal base **138** and is also greater than the stiffness of the right gluteal base **158.** In yet another embodiment the pressure equalizing zone may extend beyond edges **140, 160** and into the gluteal bases. The illustrated moisture management layer **220** is confined to the planform of the sacral member, but could be present elsewhere in the occlusion zone, instead of or in addition to being present in the sacral member. In the limit the moisture management layer is present throughout the zone of occlusion.

FIGS. **52-53** show the combined moisture management and pressure equalizing layer **220** used in the strip adhesive configuration (e.g. the configuration of FIGS. **5-6**). FIGS. **54-55** show the combined moisture management and pressure equalizing layer **220** used in the strip/distributed adhesive configuration (e.g. the configuration of FIGS. **7-8**). FIGS. **56-57** show the moisture management and pressure equalizing layer **220** used in a distributed adhesive configuration (e.g. the configuration of FIGS. **10-11**). FIGS. **58-59** show the moisture management and pressure equalizing layer **220** used in the variable strength adhesive configuration (e.g. the configuration of FIGS. **11-12**). In the arrangements of FIGS. **54-59****,** the moisture management layer and the pressure equalizing layer both reside in the zone of occlusion and are both transversely between the moisture barrier layer and the adhesive layer or other connective element **120** which is illustrated as a distributed adhesive **200** in FIGS. **52-****59.**

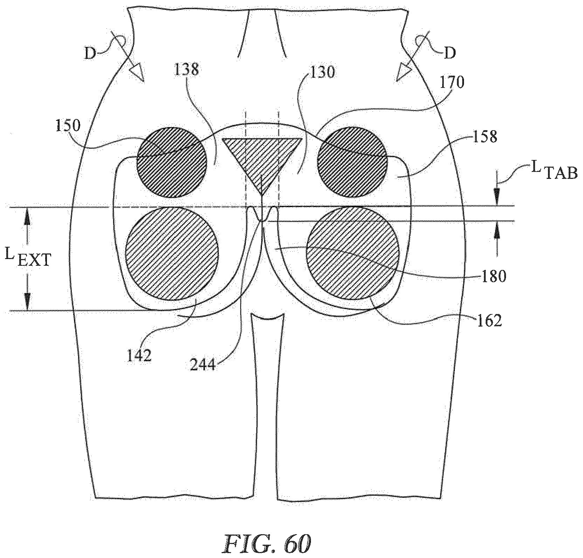

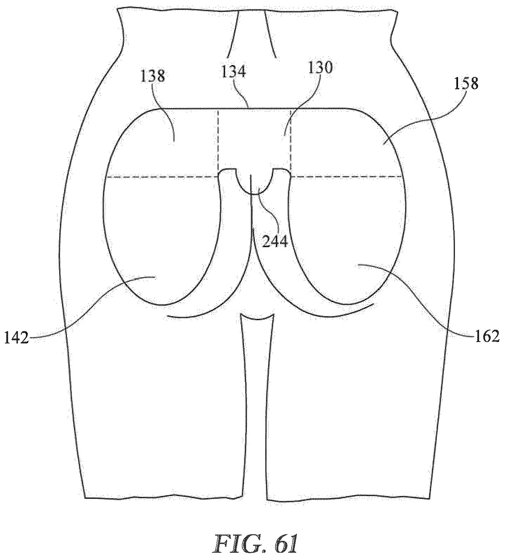

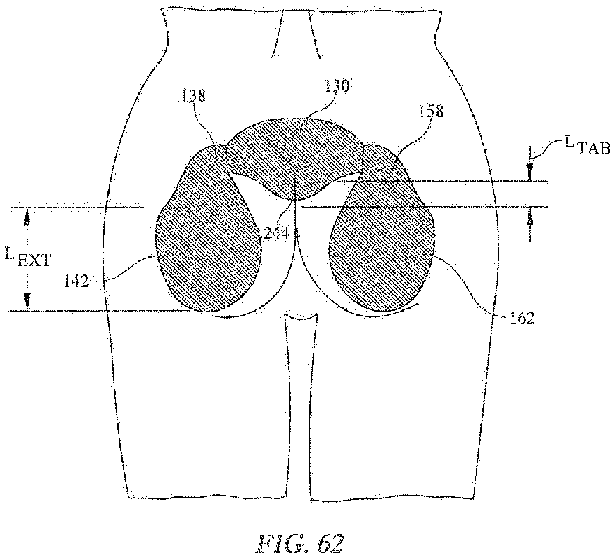

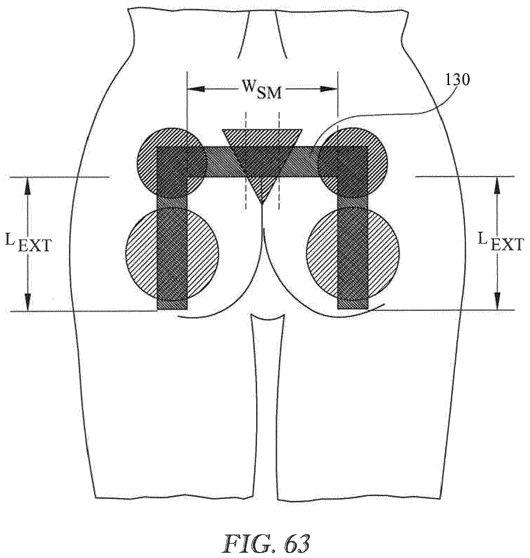

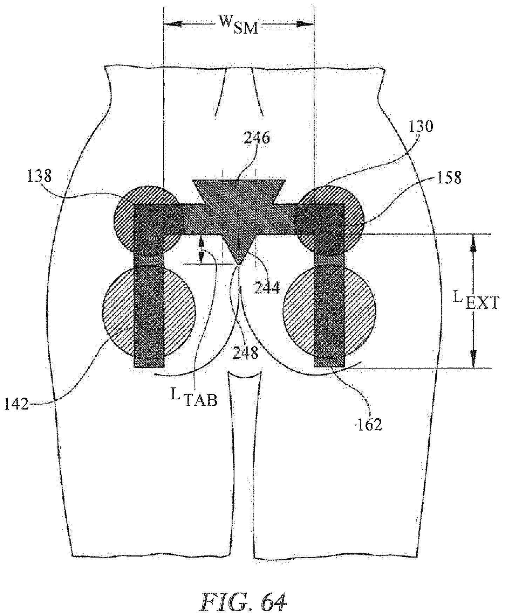

FIGS. **60-64** illustrate variations on the planform of the dressing. The planform of FIG. **60** is similar to that of FIGS. **1****,** **3A** and **4** except that edges **150, 170** of the gluteal bases are concave rather than convex from the vantage point of an observer looking in direction **D.** In addition, the dressing of FIG. **60** includes a tab **244** which extends inferiorly from the inferior edge **132** of sacral member **130** at a location between the gluteal members **136, 156.** Alternatively the tab may be thought of as a feature that defines the inferior edge and projects longitudinally into intermember space **180.** The tab has a longitudinal dimension **L_{TAB}** which is less than the longitudinal dimension **L_{EXT}** of the gluteal extensions. In particular the tab is sized and shaped so that when the dressing is applied to a care recipient the tab conforms to and nests in the care recipient's intergluteal cleft. This helps ensure a tight seal which, in turn, helps ensure that the occlusive nature of the zone of occlusion is not compromised. The dressing planform of FIG. **61** is linear along the superior edge **134** of sacral member **130.** The dressing illustrated in FIG. **62** is more irregularly shaped. The dressing illustrated in FIG. **63** has straight line borders which meet at right angles. In addition sacral member **130** is more laterally elongated than is the case in other embodiments previously described. The dressing illustrated in FIG. **64** is similar to that of FIG. **63** except at least part of the sacral member has a shape which approximates the shape of a triangle. The triangle has a base **246** corresponding approximately to the care recipient's sacral base and an apex **248.** The base is superior of the apex.

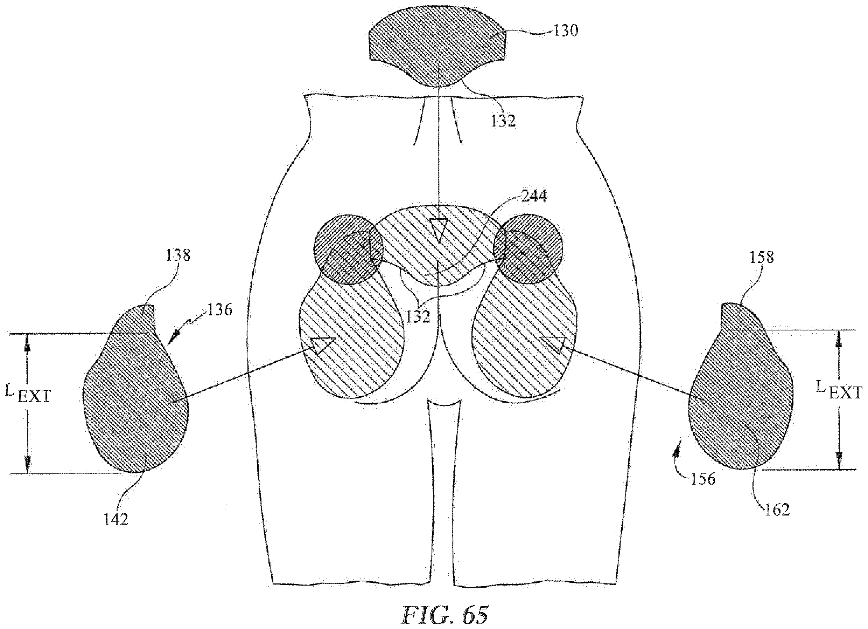

In the arrangement of FIG. **65** sacral member **130,** left gluteal member **136** and right gluteal member **156** are individual members rather than being integrated into a single unit. The left and right gluteal members are dimensioned such that gluteal extensions **142, 162** will extend inferiorly of the inferior edge **132** of the sacral member when the gluteal members are applied to a care recipient such that the gluteal members border the sacral member and the sacral member is longitudinally aligned with the care recipient's sacrum.

Although the dressing has been defined in geometric terms, it can also be described in relation to a target anatomy. In practice the dressing may be sized and proportioned for a target anatomy representative of a selected population for example a 50th percentile United States male, with the understanding that the dressing may prove to be suboptimally sized for other individuals, particularly an individual whose anatomy differs markedly from the target anatomy, for example a 1st or 99th percentile United States male. Alternatively the dressing may be sized and proportioned for an extreme target anatomy such as the 99th (or 1st) percentile anatomy, with the understanding that an oversized (or undersized) dressing may nevertheless work well for at least some range of lower (or higher) percentile individuals.

Referring again to FIGS. **1-2** and **3C** the dressing may be sized and proportioned according to the criteria set forth in table **1** below. In the table, **W_{S,IT}** is the lateral dimension of the intermember space **180** taken at a longitudinal position corresponding to the longitudinal position of the ITs of a target anatomy when the dressing is applied to the anatomy with its superior edge **134** approximately longitudinally aligned with the sacral base of the anatomy. **L_{GM}** is the longitudinal dimension of the left and right gluteal members:

**Table 1**

| dimension | value |
|---|---|
| **W_{S,IT}** | less than distance **W_{IT}** between left and right ischial tuberosity of the target anatomy. |
| **L_{GM}** | greater than or equal to a longitudinal distance **L_{L5S1-IT}** from the ischial tuberosity to the L5/S1 interface of the target anatomy. |

The distance **W_{S,IT}** in the table ensures that gluteal extensions **142, 162** are wide enough that their inner edges **144, 164** are closer to the care recipient's saggital plane **P_{SAG}** than the ITs are. As a result the gluteal extensions will extend laterally far enough toward the saggital plane to overlie the ITs. The distance **L_{GM}** ensures that the dressing extends longitudinally far enough to provide protection to the care recipient's sacral region and to the region susceptible to pressure ulcers in the vicinity of the ITs.

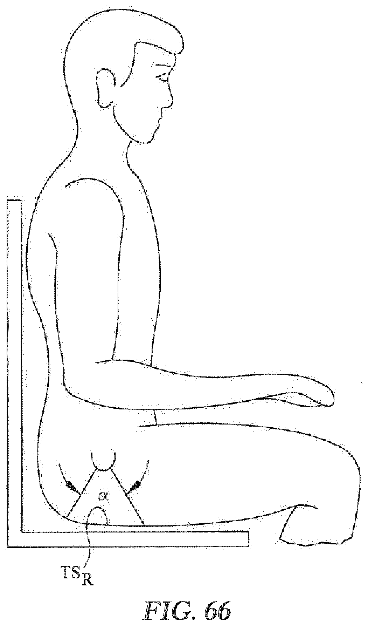

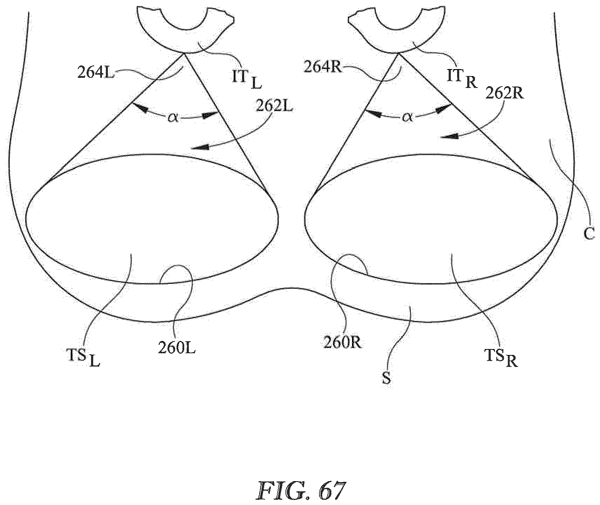

Referring additionally to FIGS. **66-67****,** according to another set of criteria the gluteal extensions are sized so that when the dressing is applied to a care recipient having a target anatomy with the superior edge **134** of sacral member **130** approximately longitudinally aligned with the care recipient's sacral base, the left and right gluteal extensions overlie respective left and right threat-susceptible regions **TS_{L}**, **TS_{R}** of the target anatomy. The left threat-susceptible region **TS_{L}** is a region bounded by the intersection **260L,** when the target anatomy is in a seated posture, of the care recipient's skin **S** and a left notional cone **262L** having a vertex **264L** at the care recipient's left ischeal tuberosity **IT_{L}** and an opening angle **α** of about 90 degrees. The right threat-susceptible region **TS_{R}** is a region bounded by the intersection **260R,** when the target anatomy is in a seated posture, of the care recipient's skin **S** and a right notional cone **262R** whose vertex is at the care recipient's right ischeal tuberosity **IT_{R}** and which has an opening angle **α** of about 90 degrees.

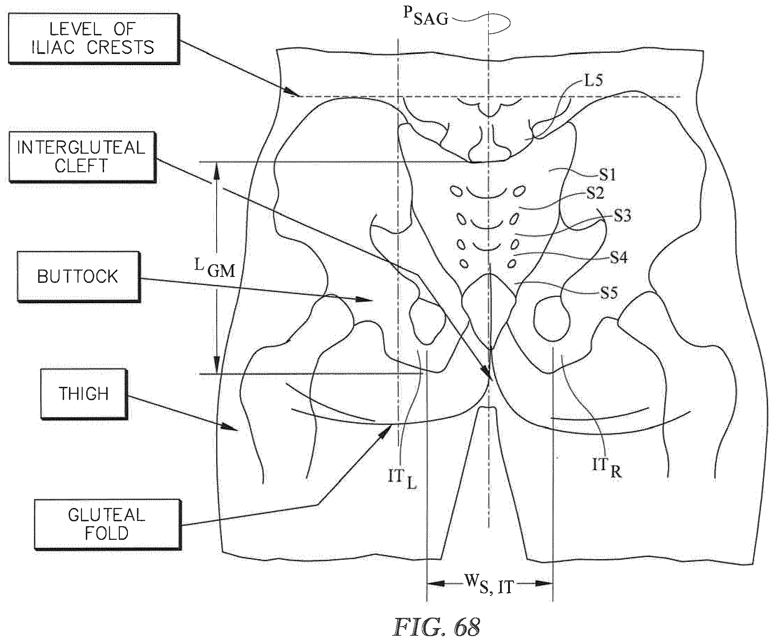

Referring to FIG. **68****,** according to another set of criteria the dressing is configured for a target adult care recipient according to the criteria set forth in table **2** below:

**Table 2**

| Dimension: | Value: |
|---|---|
| gluteal member longitudinal length **L_{GM}** | sufficiently long to extend longitudinally from the sacral base to the ischeal tuberosity. |
| gluteal member intermember distance **W_{S,IT}** | laterally narrow enough that when each gluteal member is approximately laterally centered on a corresponding gluteus maximus of the care recipient, inner edges **144, 164** of the gluteal members are, at a longitudinal position corresponding to the longitudinal position of the ITs, at least as laterally close to the saggital plane as the corresponding ischeal tuberosities are. |

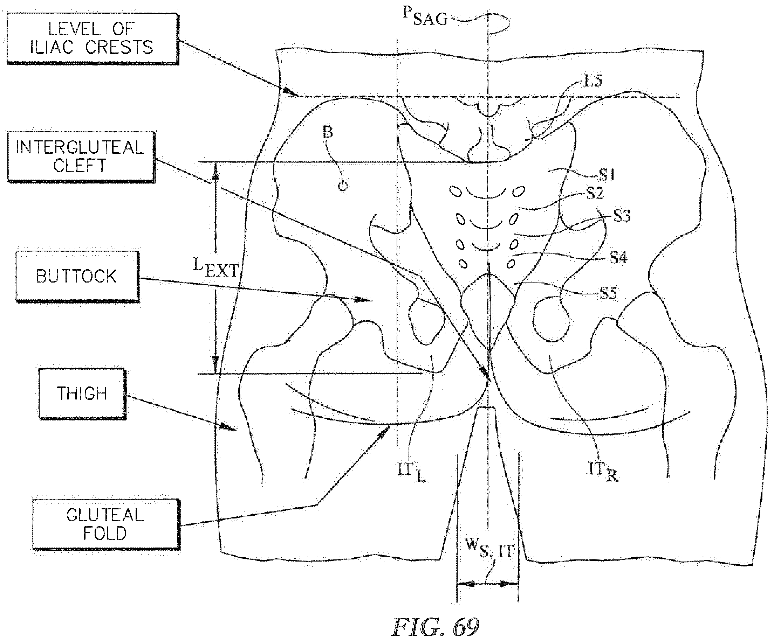

Referring to FIG. **69****,** according to another set of criteria the dressing is configured for a representative adult care recipient according to the criteria set forth in table **3** below:

**Table 3**

| Dimension: | Value: |
|---|---|
| longitudinal length **L_{EXT}** of the left and right gluteal extensions | sufficiently long to extend longitudinally from the lateral middle **"B"** of the superior edge of the respective left or right gluteus maximus to the ischeal tuberosity. |
| gluteal member intermember distance **W_{S,IT}** | laterally narrow enough that when each gluteal member is approximately laterally centered on a corresponding gluteus maximus of the care recipient, inner edges **144, 164** of the gluteal members are, at a longitudinal position corresponding to the longitudinal position of the ITs, at least as laterally close to the saggital plane as the corresponding ischeal tuberosities are. |

In yet another embodiment the preventive dressing includes a moisture barrier layer **110** and a connective element **120** on the recipient side of the dressing. As in the previously described embodiments the connective element is arranged to provide a zone of occlusion **Z.** The moisture barrier layer includes a sacral member **130** and a left gluteal member **136.** The left gluteal member has a left base **138** which borders a left side of the sacral member and a left extension **142** which extends longitudinally inferior of the left base. The moisture barrier layer also includes a right gluteal member **156.** The right gluteal member has a right base **158** which borders a right side of the sacral member and a right extension **162** which extends longitudinally inferior of the right base. The sacral member is shaped and dimensioned to overlie the sacrum of a target care recipient. The gluteal members are shaped and dimensioned to overlie the gluteus maximus muscles of the care recipient and to extend longitudinally inferiorly at least as far as the ischeal tuberosities of the care recipient when the dressing is applied to the care recipient with the sacral member approximately laterally and longitudinally aligned with the care recipient's sacrum. The dressing may also include a tab **244** which extends inferiorly from an inferior edge of the sacral member at a location between the gluteal members. The tab is sized and shaped to conform to the intergluteal cleft of the care recipient.

Although the anatomically based criteria set forth above have, in some cases, been presented separately from the geometric criteria, they do not necessarily conflict with each other. Therefore a designer may find it useful to choose design criteria from among the separately enumerated criteria.

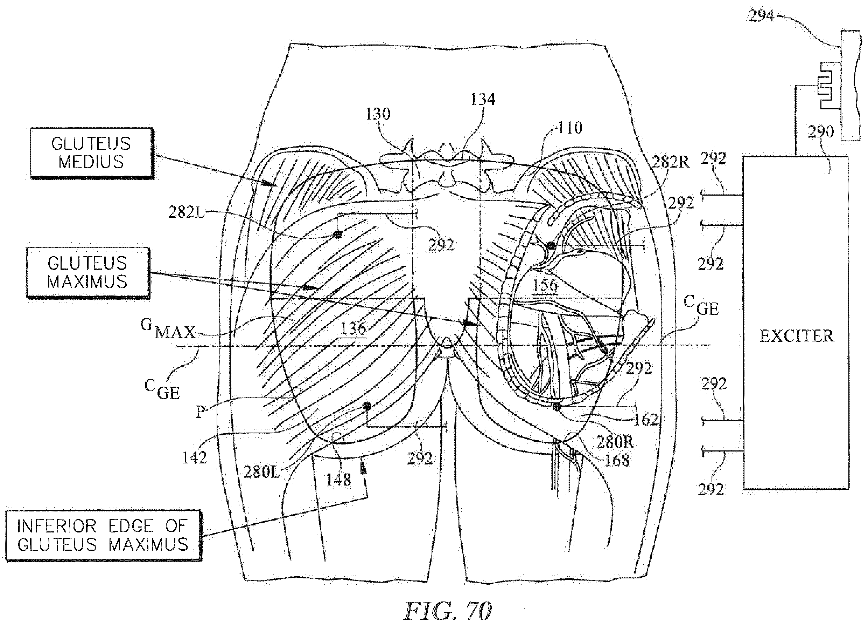

FIG. **70** shows a preventive dressing assembly **270** which comprises a dressing **100** and an exciter **280.** The dressing of the dressing assembly, like the dressings already described, has a moisture barrier layer **110** with a recipient side, an environmental side, a superior edge **134,** an inferior edge defined by gluteal extension inferior edges **148, 168,** a perimeter **P,** and a connective element, such as an adhesive, on the recipient side of the moisture barrier layer.

Unlike the dressings already described the dressing **100** of the dressing assembly **270** also includes an array of electrodes. The electrode array includes a left inferior electrode **280L,** a left intermediate electrode **282L,** a right inferior electrode **280R,** and a right intermediate electrode **282R.** The intermediate electrodes are superior of the inferior electrodes. In the illustrated embodiment the electrodes are positioned relative to each other within dressing perimeter **P** so that the left electrodes overlie the left gluteus maximus of a care recipient and the right electrodes overlie the right gluteus maximus of the care recipient when the dressing is applied to the care recipient with sacral member **130** overlying the care recipient's sacrum. In the illustrated embodiment the intermediate electrodes **282** are longitudinally aligned with sacral member **130,** and the inferior electrodes **280** are positioned near the termini **148, 168** of the gluteal extensions, for example inferior of a laterally extending centerlines **C_{GE}** of the gluteal members but nevertheless not where they would be inferior of the inferior edge of the gluteus maximus of the care recipient.

The dressing assembly also includes an exciter **290** connected to the electrodes by wires **292.** The illustrated exciter is connected to an AC power source **294** such as a conventional outlet. Alternatively, power source **294** may be an RF power source or a near field power source. The exciter is adapted to selectively apply an intermittent excitation to specific electrodes, for example a left excitation to only the left electrodes, a right excitation to only the right electrodes, concurrent excitation to both the left electrodes and the right electrodes, and excitation to longitudinally aligned electrodes for example to the left and right intermediate electrodes. An example excitation is the application of a voltage difference across two electrodes.

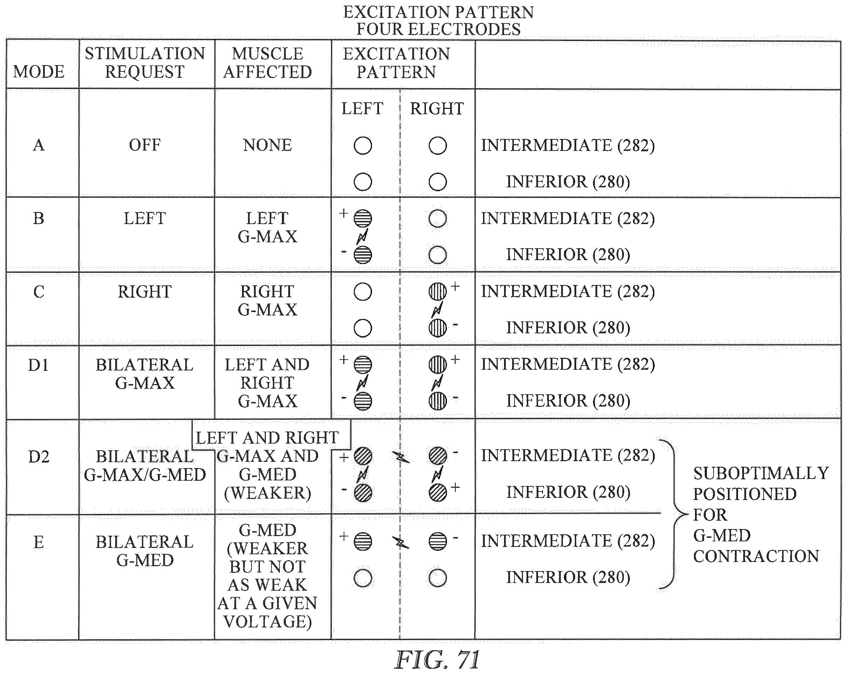

In the illustrated embodiment the left intermediate electrode **282L** is laterally aligned with the left gluteal member **136** and the right intermediate electrode **282R** is laterally aligned with the right gluteal member **156.** The left inferior electrode **280L** is on the left gluteal extension **142** longitudinally inferior of the left intermediate electrode, and the right inferior electrode **280R** is on the right gluteal extension **162** inferior of the right intermediate electrode. Referring additionally to FIG. **71****,** exciter **290** is capable of selectively applying an excitation in each excitation mode set forth in table **4** below. A lightning bolt symbol in FIG. **71** indicates which electrodes are companions of each other for each mode of excitation. Plus and minus signs next to the electrode symbols show voltage polarity (plus for higher voltage; minus for lower voltage) for each companion pair.

**Table 4**

| Mode | Involved Electrodes | Relative Polarity |
|---|---|---|
| A | none | not applicable |
| B | left intermediate / left inferior | either |
| C | right intermediate / right inferior | either |
| D1 | left intermediate / left inferior and right intermediate / right inferior | same left and right |
| D2 | left intermediate / left inferior and right intermediate / right inferior | opposite left and right |
| E | left intermediate / right intermediate | either |

The dressing is sized and the electrodes are positioned so that when the dressing is applied to a care recipient having a target anatomy with each gluteal member approximately laterally centered on a corresponding gluteus maximus of the care recipient, and the sacral member approximately laterally and longitudinally aligned with the sacrum of the care recipient, the excitation stimulates one or more muscles of the care recipient as set forth in table **5** below and in FIG. **71****.**

**Table 5**

| Mode | Involved Electrodes | Relative Polarity | Primary Muscle Stimulated | Secondary Muscle Stimulated |
|---|---|---|---|---|
| A | none | not applicable | none | none |
| B | left intermediate / left inferior | either | Left gluteus maximus | |
| C | right intermediate / right inferior | either | Right gluteus maximus | |
| D1 | left intermediate / left inferior and right intermediate / right inferior | same left and right | Left gluteus maximus and right gluteus maximus | |
| D2 | left intermediate / left inferior and right intermediate / right inferior | opposite left and right | Left gluteus maximus and right gluteus maximus. | superior region of left and right gluteus medius (weak) |
| E | left intermediate / right intermediate | either | superior region of left and right gluteus medius (not as weak as mode D2 for a given voltage) | |

Modes D1 and D2 are the same except for the voltage polarity. In mode D1 electrical current flows through the gluteal muscle tissue between left intermediate electrode **282L** and left inferior electrode **280L** thereby stimulating the left gluteus maximus of the care recipient. Similarly, electrical current flows through the gluteal muscle tissue between right intermediate electrode **182R** and right inferior electrode **280R** thereby stimulating the right gluteus maximus. The excitation causes the muscle to contract which makes the tissue less susceptible to deep tissue injury. Because the voltage at both intermediate electrodes is the higher voltage, there is no noteworthy current flow laterally between the left and right intermediate electrodes.

In mode D2 electrical current flows longitudinally between left intermediate electrode **282L** and left inferior electrode **280L** by way of the left gluteal muscle tissue thereby stimulating the left gluteus maximus. Electrical current flows longitudinally between right intermediate electrode **282R** and right inferior electrode **280R** by way of the right gluteal muscle tissue thereby stimulating the right gluteus maximus. In addition, the voltage difference between the left and right intermediate electrodes is expected to cause current to flow laterally between the left gluteus medius and the right gluteus medius thereby stimulating the gluteus medius muscles. The lateral current flow is expected to be relatively weak in comparison to the longitudinal current flow because the gluteus maximus muscles lie transversely between the intermediate electrodes and the gluteus medius muscles. The electrodes are therefore not optimally positioned to cause lateral current flow.

In mode E electrical current flows between the left and right intermediate electrodes thereby stimulating the left and right gluteus medius muscles. As with mode D2 the electrodes are not optimally positioned to stimulate the gluteus medius, however the stimulation is expected to be less weak than that of mode D2.

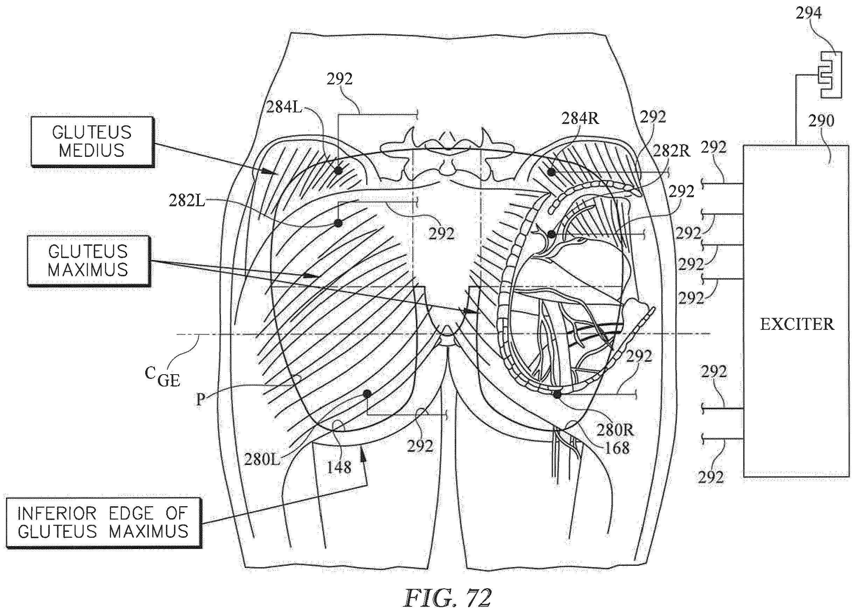

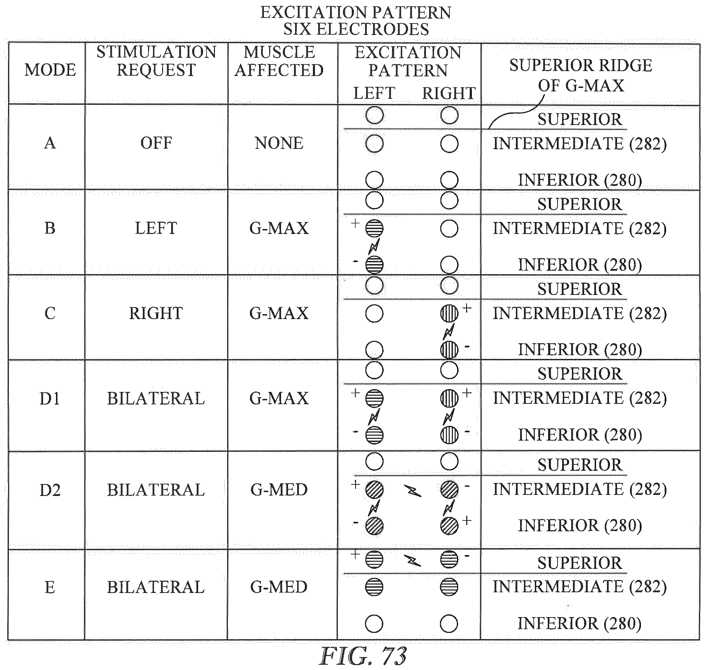

The foregoing describes a four electrode embodiment. FIGS. **72-73** shows a six electrode embodiment. In the diagrams of FIG. **73** a horizontal line schematically signifies the superior edge of the gluteus maximus and therefore distinguishes between the electrodes that are superior of and inferior of the superior edge of the gluteus maximus. The six electrode embodiment includes a left superior electrode **284L** longitudinally superior of left intermediate electrode **282L** and a right superior electrode **284R** longitudinally superior of the right intermediate electrode **282R.** In the illustrated embodiment the electrodes are positioned relative to each other within dressing perimeter **P,** so that the left and right superior electrodes overlie the left and right gluteus medius muscles of a care recipient when the dressing is applied to the care recipient with sacral member **130** overlying the care recipient's sacrum. In the illustrated embodiment the superior electrodes **284** are longitudinally aligned with sacral member **130,** and the inferior electrodes **280** are positioned near the termini of the gluteal extensions, for example inferior of laterally extending centerlines **C_{GE}** of the gluteal members but nevertheless not where they would be inferior of the edge of the gluteus maximus of the care recipient.

The dressing assembly also includes an exciter **290** connected to the electrodes by wires **292.** The illustrated exciter is connected to an AC power source **294** such as a conventional outlet. Alternatively, power source **294** may be an RF power source or a near field power source. The exciter is adapted to selectively apply an excitation to specific electrodes, for example a left excitation to only the left electrodes, a right excitation to only the right electrodes, concurrent excitation to both the left electrodes and the right electrodes, and excitation to longitudinally aligned electrodes for example to the left and right superior electrodes. An example excitation is the application of a voltage difference across two electrodes.

In the illustrated embodiment the left superior electrode **284L** is laterally aligned with left gluteal member **136** and the right superior electrode **284R** is laterally aligned with the right gluteal member **156.** The left inferior electrode **280L** is on the left gluteal extension **142** longitudinally inferior of the left intermediate electrode **282L,** and the right inferior electrode **280R** is on the right gluteal extension **162** longitudinally inferior of the right intermediate electrode. Exciter **290** is capable of selectively applying an excitation in each excitation mode set forth in table **7** below. A lightning bolt symbol in FIG. **73** indicates which electrodes are companions of each other for each mode of excitation. Plus and minus signs next to the electrode symbols show voltage polarity (plus for higher voltage; minus for lower voltage) for each companion pair.

**Table 7**

| Mode | Involved Electrodes | Relative Polarity |
|---|---|---|
| A | none | either |
| B | left intermediate / left inferior | either |
| C | right intermediate / right inferior | either |
| D1 | left intermediate / left inferior and right intermediate / right inferior | same left and right |
| D2 | left intermediate / left inferior and right intermediate / right inferior | opposite left and right |
| E | left superior / right superior | either |

The dressing is sized and the electrodes are positioned so that when the dressing is applied to a care recipient having a target anatomy with each gluteal member approximately laterally centered on a corresponding gluteus maximus of the care recipient, and the sacral member approximately laterally and longitudinally aligned with the sacrum of the care recipient, the excitation stimulates one or more muscles of the care recipient as set forth in table **8** below and in FIG. **73****.**

**Table 8**

| Mode | Involved Electrodes | Relative Polarity | Primary Muscle Stimulated | Secondary Muscle Stimulated |
|---|---|---|---|---|
| A | none | not applicable | none | none |
| B | left intermediate / left inferior | either | Left gluteus maximus | |
| C | right intermediate / right inferior | either | Right gluteus maximus | |
| D1 | left intermediate / left inferior and right intermediate / right inferior | same left and right | Left gluteus maximus and right gluteus maximus | |
| D2 | left intermediate / left inferior and right intermediate/ right inferior | opposite left and right | Left gluteus maximus and right gluteus maximus | superior region of left and right gluteus medius (weak) |
| E | left superior / right superior | either | superior region of left and right gluteus medius | |

Modes D1 and D2 are the same except for the voltage polarity. In mode D1 electrical current flows between the left intermediate electrode **282L** and the left inferior electrode **280L** by way of the left gluteus maximus thereby stimulating the left gluteus maximus. Similarly, electrical current flows from right intermediate electrode **282R** to the right inferior electrode **280R** by way of the right gluteus maximus thereby stimulating the right gluteus maximus. The excitation causes the gluteus maximus muscles to contract which makes the tissue less susceptible to deep tissue injury. Because the voltage at both intermediate electrodes is the higher voltage, there is no noteworthy current flow laterally between the left and right intermediate electrodes.

In mode D2 electrical current flows through the left gluteus maximus between left intermediate electrode **282L** and left inferior electrode **280L** thereby stimulating the left gluteus maximus. Electrical current flows through the right gluteus maximus between the right intermediate electrode **282R** and the right inferior electrode **280R** thereby stimulating the right gluteus maximus. In addition, the voltage difference between the left and right intermediate electrodes is expected to cause current to flow laterally between the left gluteus medius and the right gluteus medius thereby stimulating the gluteus medius muscles. The lateral current flow is expected to be relatively weak in comparison to the longitudinal current flow because the gluteus maximus muscles lie transversely between the intermediate electrodes and the gluteus medius muscles. The electrodes are therefore not optimally positioned to cause lateral current flow.

In mode E electrical current flows between the left and right superior electrodes thereby stimulating the left and right gluteus medius muscles.

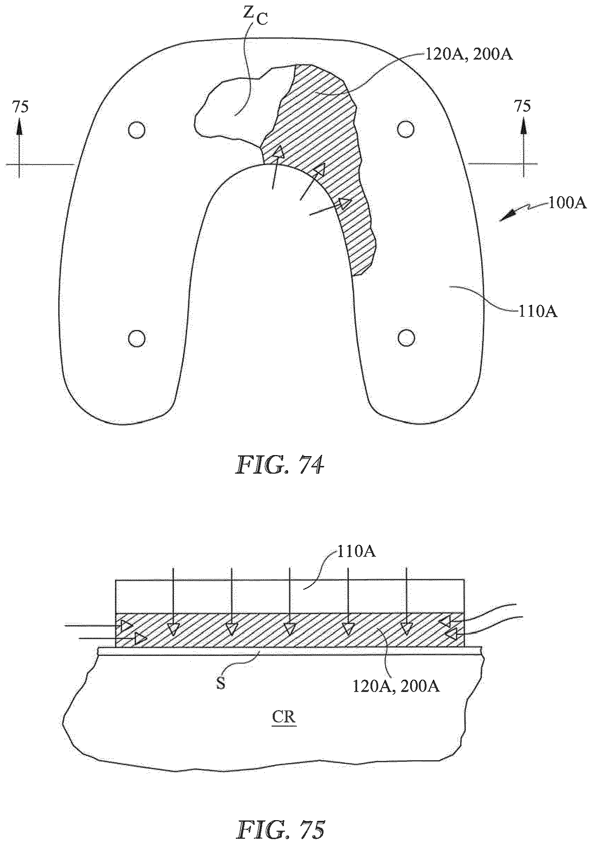

In yet another set of embodiments the electrode arrangements and electrode excitation patterns are as already described in connection with FIGS. **70-73****,** however other elements of the dressing do not cooperate to provide a zone of occlusion. Referring to FIGS. **74-75****,** a dressing **100A** includes an external layer **110A** which is analogous to moisture barrier layer **110** as previously described but which does not prevent moisture transfer from the environment to the portion of the care recipient's skin covered by the dressing. In addition, connective element **120A** (illustrated as a spatially distributed adhesive **200A)** is an adhesive element which also does not protect against moisture ingress. As a result, the region of the care recipient's anatomy covered by the dressing is a zone of coverage **Z_{c}** rather than a zone of occlusion **Z.** In such an embodiment the dressing serves as an electrode host which is configured to place the electrodes at the desired location on the anatomy of the care recipient, but does not protect against moisture ingress into the area covered by the dressing (i.e. into zone **Z_{c}**). This is indicated by the solid arrows which penetrate transversely through external layer **110A** and connective element **120A** and which also penetrate longitudinally and laterally into connective element **120A.** FIG. **74** shows the four electrode variant already described in connection with FIGS. **70-71****,** however dressing **100A** can also accommodate the six electrode variant of FIGS. **72-73****.**

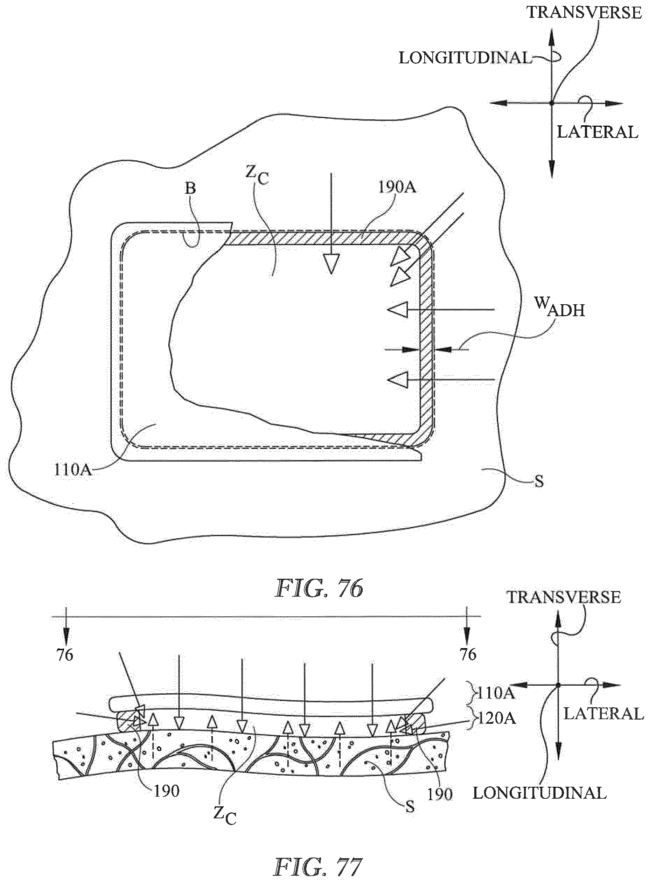

FIGS. **76-77** show an embodiment similar to that of FIGS. **74-75** but in which the connective element **120A** is illustrated as an adhesive strip **190A.** Moisture transfer is indicated by the solid arrows which penetrate transversely through external layer **110A** and which also penetrate longitudinally and laterally through adhesive strip **190A.** Once again the dressing serves as an electrode host which is configured to place the electrodes at the desired location on the anatomy of the care recipient but which does not protect against moisture ingress transversely across external layer **110A** or laterally and longitudinally through adhesive strip **190A.**

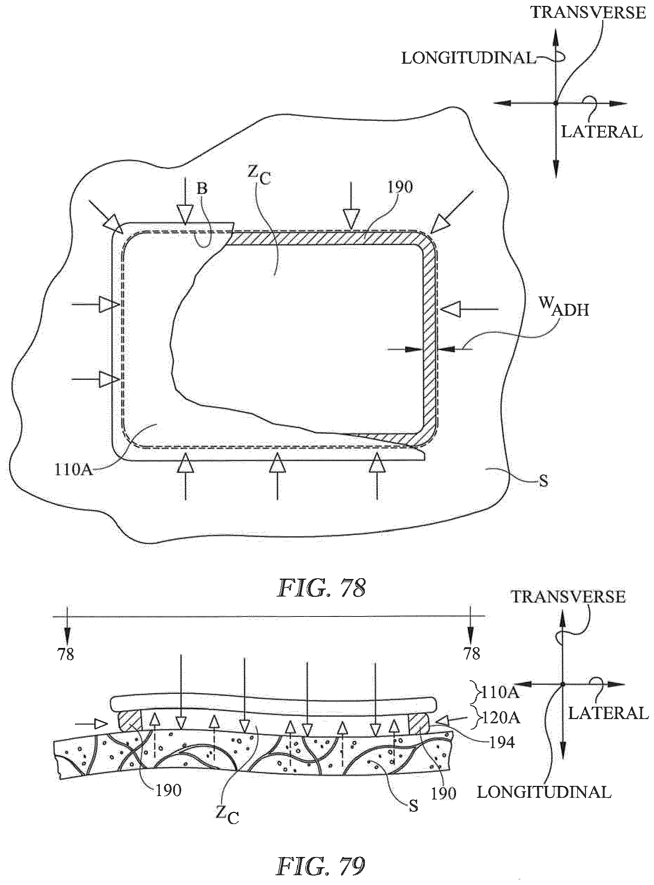

FIGS. **78-79** show an embodiment similar to that of FIGS. **76-77** but in which the connective element **120** is a an adhesive strip **190** which blocks moisture transfer laterally and longitudinally into zone **Z_{c}**. Moisture can nevertheless enter zone **Z_{c}** by way of external layer **110A**. This is indicated by the solid arrows which penetrate transversely through external layer **110A** and by the laterally and longitudinally directed solid arrows which terminate without crossing adhesive strip **190.** Once again the dressing serves as an electrode host which is configured to place the electrodes at the desired location on the anatomy of the care recipient but which does not protect against moisture ingress transversely across external layer **110A**.

The variations in planform, such as those of FIGS. **60-65****,** apply equally to the non-occlusive embodiments just described. In addition a pressure equalizing layer such as layer **220** described in connection with FIGS. **43-50** may also be incorporated.

Although this disclosure refers to specific embodiments, it will be understood by those skilled in the art that various changes in form and detail may be made.

Embodiments of the invention can be described with reference to the following numbered clauses, with preferred features laid out in the dependent clauses:
**1.** A preventive dressing having a recipient side, an environmental side, and a perimeter, the dressing comprising:
A) a moisture barrier layer having:
   a) a sacral member having a longitudinal length and an inferior edge;
   b) a left gluteal member having a base which borders a left side of the sacral member, and a left extension which extends longitudinally inferiorly further than the inferior edge by a left gluteal extension dimension which is approximately no less than the longitudinal length of the sacral member;
   c) a right gluteal member having a base which borders a right side of the sacral member, and a right extension which extends longitudinally inferiorly further than the inferior edge by a right gluteal extension dimension which is approximately no less than the longitudinal length of the sacral member so that the left gluteal member and the right gluteal member define an intermember space; and
B) a connective element on the recipient side of the dressing, the connective element being arranged to provide a zone of occlusion.

**2.** The dressing of clause **1** wherein the connective element comprises an adhesive.
**3.** The dressing of clause **2** wherein the adhesive is one-way liquid absorbant.
**4.** The dressing of clause **2** wherein the adhesive is one-way liquid permeable.
**5.** The dressing of clause **1** wherein the connective element is a continuous strip of adhesive extending along substantially the perimeter of the dressing.
**6.** The dressing of clause **5** wherein the adhesive strip borders the occlusion zone and wherein an auxiliary layer resides in the occlusion zone, and the auxiliary layer comprises at least one of:
a liquid absorbant layer,
a liquid permeable layer,
a pressure equalizing layer.

**7.** The dressing of clause **1** wherein the connective element comprises a sacral adhesive layer and a gluteal adhesive element and wherein:
the sacral adhesive layer is an adhesive which forms substantially all of:
   a) the recipient side of the sacral member, and
   b) the recipient sides of the left and right gluteal bases,
the gluteal extensions have a margin and the gluteal adhesive element is a continuous strip of adhesive extending along substantially the margin of the gluteal member extensions.

**8.** The dressing of clause 7 including at least one auxiliary layer residing in the zone of occlusion.
**9.** The dressing of clause **8** wherein the auxiliary layer comprises at least one of:
A) a liquid absorbant layer,
B) a liquid permeable layer, and
C) a pressure equalizing layer.

**10.** The dressing of clause 1 wherein the connective element is an adhesive which forms substantially all of the recipient side of the dressing.
**11.** The dressing of clause **10** including at least one auxiliary layer residing in the zone of occlusion.
**12.** The dressing of clause **11** wherein the auxiliary layer comprises at least one of:
A) a liquid absorbant layer,
B) a liquid permeable layer, and
C) a pressure equalizing layer.

**13.** The dressing of clause **10** wherein the connective element comprises a first adhesive composition forming substantially all of the recipient side of the sacral member and the gluteal bases, and a second adhesive composition forming substantially all of the recipient side of the gluteal extensions and wherein the first adhesive composition adheres relatively more strongly to human skin and the second adhesive composition adheres relatively more weakly to human skin.
**14.** The dressing of clause **1** including a pressure equalizing layer spanning laterally across a longitudinally extending centerline of the dressing in the zone of occlusion to define a pressure equalizing zone, the pressure equalizing layer imparting a stiffness to the pressure equalizing zone which is greater than the stiffness of the dressing laterally to the left and right of the pressure equalizing zone.
**15.** The dressing of clause **1** including a pressure equalizing zone spanning laterally across a longitudinally extending centerline of the dressing in the zone of occlusion, the pressure equalizing zone having a planform and a thickness whick is greater than the thickness of the dressing outside the planform of the pressure equalizing zone.
**16.** The dressing of clause **1** including a zone spanning laterally across a longitudinally extending centerline of the dressing in the zone of occlusion, the zone having a customizable thickness.
**17.** The dressing of clause **16** wherein the customizable thickness of the zone of customizable thickness is attributable to peel-away sublayers.
**18.** The dressing of clause **1** including a classified zone spanning laterally across a longitudinally extending centerline of the dressing in the zone of occlusion, the classified zone having a class specific thickness which is one of a set of two or more class specific thicknesses.
**19.** The dressing of clause **14** wherein the pressure equalizing layer is confined to the sacral member.
**20.** The dressing of clause **1** including a moisture management layer in the zone of occlusion.
**21.** the dresssing of clause **20** wherein the moisture management layer is a liquid absorbant layer.
**22.** the dresssing of clause **20** wherein the moisture management layer is a liquid permeable layer.
**23.** The dressing of clause 1 including a moisture management layer and a pressure equalizing layer both of which are in the zone of occlusion and transversely between the moisture barrier layer and the connective element.
**24.** The dressing of clause **23** wherein the pressure equalizing layer is confined to the sacral member.
**25.** The dressing of clause **1** comprising a tab which extends inferiorly from the sacral member at a location between the gluteal members, the tab having a longitudinal dimension which is less than the longitudinal dimension of the extensions.
**26.** The dressing of clause **1** wherein dimensions of the dressing are as set forth in the table below in which
**W_{S,IT}** is a lateral dimension of the intermember space taken at a longitudinal position corresponding to the longitudinal position of the ITs of a target anatomy when the dressing is applied to the anatomy with its superior edge approximately longitudinally aligned with the sacral base of the anatomy and
**L_{GM}** is the longitudinal dimension of the left and right gluteal members:

| dimension | value |
|---|---|
| **W_{S,IT}** | less than distance between left and right ischial tuberosity of the target anatomy. |
| **L_{GM}** | greater than or equal to a longitudinal distance **L_{L5S1-IT}** from the ischial tuberosity to the L5/S1 interface of the target anatomy. |

**27.** The dressing of clause **26** wherein the target anatomy is that of a 50th percentile United States male.
**28.** The dressing of clause **1** wherein the gluteal extensions are sized so that when the dressing is applied to a care recipient having a target anatomy comprising a skin, a left ischeal tuberosity and a right ischeal tuberosity so that its superior edge is approximately longitudinally aligned with the care recipient's sacral base:
the left gluteal extension covers a left threat-susceptible region of the target anatomy, the left target region being the intersection of the skin and a left notional cone when the target anatomy is in a seated posture, the left cone having a vertex at the left ischeal tuberosity and an opening angle of about **90** degrees, and
the right gluteal extension covers a right threat-susceptible region of the target anatomy, the right target region being the intersection of the skin and a right notional cone when the target anatomy is in a seated posture, the right cone having a vertex at the right ischeal tuberosity and an opening angle of about **90** degrees.

**29.** The dressing of clause **1** comprising a tab which extends inferiorly from the sacral member at a location between the gluteal members, the tab being sized and shaped to conform to the intergluteal cleft of a care recipient thereby preserving occlusivity of the zone of occlusion.
**30.** The dressing of clause **1** wherein at least part of the sacral member has a shape which approximates the shape of a triangle having a base and an apex, the base being superior of the apex.
**31.** The dressing of clause **1** wherein the sacral member, the left gluteal member and the right gluteal member are individual members and wherein the left and right gluteal members are dimensioned such that the extensions thereof extend inferiorly of the inferior edge of the sacral member when the gluteal members are applied to a care recipient such that the gluteal members border the sacral member and the sacral member is applied to the care recipient the sacral member **130** is longitudinally aligned with the care recipient's sacrum.
**32.** The dressing of clause **1** wherein the dressing is configured for a target adult care recipient having a sacrum, a sacral base, a left gluteus maximus having a superior edge, a right gluteus maximus having a superior edge, a left ischeal tuberosity, and a right ischeal tuberosity as set forth below:

| Dimension: | Value: |
|---|---|
| gluteal member longitudinal length **L_{GM}** | sufficiently long to extend longitudinally from the sacral base to the ischeal tuberosity. |
| gluteal member intermember distance **W_{S,IT}** | laterally narrow enough that when each gluteal member is approximately laterally centered on a corresponding gluteus maximus of the care recipient, inner edges of the gluteal members are, at a longitudinal position corresponding to the longitudinal position of the ITs, at least as laterally close to the saggital plane as the corresponding ischeal tuberosities are. |

**33.** The dressing of clause **1** wherein the dressing is configured for a target adult care recipient having a sacrum, a sacral base, a left gluteus maximus, a right gluteus maximus, a left ischeal tuberosity, and a right ischeal tuberosity as set forth below:

| Dimension: | Value: |
|---|---|
| gluteal extension longitudinal length **L_{EXT}** | sufficiently long to extend longitudinally from the lateral middle of the superior edge of the gluteus maximus to the ischeal tuberosity. |
| gluteal member intermember distance **W_{S,IT}** | laterally narrow enough that when each gluteal member is approximately laterally centered on a corresponding gluteus maximus of the care recipient, inner edges of the gluteal members are, at a longitudinal position corresponding to the longitudinal position of the ITs, at least as laterally close to the saggital plane as the corresponding ischeal tuberosiies are. |

**34.** A preventive dressing having a recipient side and an environmental side, the dressing comprising:
A) a moisture barrier layer having:
   a) a sacral member
   b) a left gluteal member having a left base which borders a left side of the sacral member, and a left extension which extends longitudinally inferior of the left base,
   c) a right gluteal member having a right base which borders a right side of the sacral member, and a right extension which extends longitudinally inferior of the right base;
   the sacral member being shaped and dimensioned to overlie the sacrum of a target care recipient, and the gluteal members being shaped and dimensioned to overlie the gluteus maximus muscles of the care recipient and to extend longitudinally inferiorly at least as far as the ischeal tuberosities of the care recipient when the dressing is applied to the care recipient with the sacral member approximately laterally and longitudinally aligned with the care recipient's sacrum; and
B) a connective element on the recipient side of the dressing, the connective element being arranged to provide a zone of occlusion.

**35.** The dressing of clause **34** comprising a tab which extends inferiorly from an inferior edge of the sacral member at a location between the gluteal members, the tab being sized and shaped to conform to the intergluteal cleft of the care recipient thereby preserving occlusivity of the zone of occlusion.
**36.** The dressing of clause **1** comprising:
a left intermediate electrode laterally aligned with the left gluteal member;
a right intermediate electrode laterally aligned with the right gluteal member;
a left inferior electrode on the left gluteal extension longitudinally inferior of the left intermediate electrode; and
a right inferior electrode on the right gluteal extension longitudinally inferior of the right intermediate electrode.

**37.** The dressing of clause **36** including an exciter capable of selectively applying an intermittent excitation in each excitation mode set forth below:

| Mode | Involved Electrodes | Relative Polarity |
|---|---|---|
| A | none | not applicable |
| B | left intermediate / left inferior | either |
| C | right intermediate / right inferior | either |
| D1 | left intermediate / left inferior and right intermediate / right inferior | same left and right |
| D2 | left intermediate / left inferior and right intermediate / right inferior | opposite left and right |
| E | left intermediate / right intermediate | either |

**38.** The dressing of clause **37** wherein the dressing is sized and the electrodes are positioned such that when the dressing is applied to a care recipient having a target anatomy with each gluteal member approximately laterally centered on a corresponding gluteus maximus of the care recipient and the sacral member approximately laterally and longitudinally aligned with the sacrum of the care recipient, the excitation stimulates one or more muscles of the care recipient as set forth below.

| Mode | Involved Electrodes | Relative Polarity | Primary Muscle Stimulated | Secondary Muscle Stimulated |
|---|---|---|---|---|
| A | none | not applicable | none | none |
| B | left intermediate / left inferior | either | Left gluteus maximus | |
| C | right intermediate / right inferior | either | Right gluteus maximus | |
| D1 | left intermediate / left inferior and right intermediate / right inferior | same left and right | Left gluteus maximus and right gluteus maximus | |
| D2 | left intermediate / left inferior and right intermediate / right inferior | opposite left and right | Left gluteus maximus and right gluteus maximus. | superior region of left and right gluteus medius (weak) |
| E | left intermediate / right intermediate | either | superior region of left and right gluteus medius (not as weak as mode D2 for a given voltage) | |

**39.** The dressing of clause **36** wherein the left and right gluteal members each have an inferior terminus, the left and right inferior electrodes are positioned near the termini of the left and right gluteal members respectively, and the left and right intermediate electrodes are positioned in approximate longitudinal alignment with the sacral member.
**40.** The dressing of clause **36** including a left superior electrode longitudinally superior of the left intermediate electrode and a right superior electrode longitudinally superior of the right intermediate electrode.
**41.** The dressing of clause **40** including an exciter capable of selectively applying an intermittent excitation in each excitation mode set forth below:

| Mode | Involved Electrodes | Relative Polarity |
|---|---|---|
| A | none | either |
| B | left intermediate / left inferior | either |
| C | right intermediate / right inferior | either |
| D1 | left intermediate / left inferior and right intermediate / right inferior | same left and right |
| D2 | left intermediate / left inferior and right intermediate / right inferior | opposite left and right |
| E | left superior / right superior | either |

**42.** The dressing of clause **41** wherein the dressing is sized such that when the dressing is applied to a care recipient having a target anatomy with each gluteal member approximately laterally centered on a corresponding gluteus maximus of the care recipient and the sacral member approximately laterally and longitudinally aligned with the sacrum of the care recipient, the excitation stimulates one or more muscles of the care recipient as set forth below.

| Mode | Involved Electrodes | Relative Polarity | Primary Muscle Stimulated | Secondary Muscle Stimulated |
|---|---|---|---|---|
| A | none | not applicable | none | none |
| B | left intermediate / left inferior | either | Left gluteus maximus | |
| C | right intermediate / right inferior | either | Right gluteus maximus | |
| D1 | left intermediate / left inferior and right intermediate / right inferior | same left and right | Left gluteus maximus and right gluteus maximus | |
| D2 | left intermediate / left inferior and right intermediate/ right inferior | opposite left and right | Left gluteus maximus and right gluteus maximus | superior region of left and right gluteus medius (weak) |
| E | left superior / right superior | either | superior region of left and right gluteus medius | |

**43.** The dressing of clause **40** wherein the left and right gluteal members each have an inferior terminus, the left and right inferior electrodes are positioned near the termini of the left and right gluteal members respectively, and the left and right superior electrodes are positioned in approximate longitudinal alignment with the sacral member.
**44.** A preventive dressing assembly comprised of:
A) a moisture barrier layer having a recipient side, an environmental side, a superior end, an inferior end, and a perimeter;
B) a left intermediate electrode and a left inferior electrode, the left intermediate electrode being superior of the left inferior electrode, the left electrodes being positioned relative to each other within the perimeter in order to overlie the left gluteus maximus of a care recipient;
C) a right intermediate electrode and a right inferior electrode, the right intermediate electrode being superior of the right inferior electrode the right electrodes being positioned relative to each other within the perimeter in order to overlie the right gluteus maximus of a care recipient;
D) a connective element on the recipient side of the moisture barrier layer.

**45.** The dressing assembly of clause **44** wherein the moisture barrier layer, the left electrodes, the right electrodes, and the connective element comprise a dressing and wherein the dressing assembly also includes an exciter connected to the electrodes, the exciter adapted to selectively and intermittently apply:
A) a left excitation to only the left electrodes,
B) a right excitation to only the right electrodes,
C) a concurrent excitation to both the left electrodes and the right electrodes, and
D) an excitation to longitudinally aligned electrodes.

**46.** The dressing assembly of clause **44** comprising a left superior electrode longitudinally superior of the left intermediate electrode, a right superior electrode longitudinally superior of the right intermediate electrode, the left and right superior electrodes being positioned relative to each other within the perimeter in order to overlie the left and right gluteus medius respectively of a care recipient.
**47.** The dressing assembly of clause **46** wherein the moisture barrier layer, the left electrodes, the right electrodes, and the connective element comprise a dressing and wherein the dressing assembly also includes an exciter connected to the electrodes, the exciter adapted to selectively and intermittently apply:
A) a left excitation to only the left intermediate and inferior electrodes;
B) a right excitation to only the right intermediate and inferior electrodes;
C) a concurrent excitation to the left intermediate and inferior electrodes and to the right intermediate and inferior electrodes;
D) an excitation to the left and right superior electrodes.

**48.** A preventive dressing having a recipient side, an environmental side, and a perimeter, the dressing comprising:
an external layer having:
a left gluteal member;
a right gluteal member;
a left intermediate electrode on the left gluteal member;
a right intermediate electrode on the right gluteal member;
a left inferior electrode on the left gluteal member longitudinally inferior of the left intermediate electrode;
a right inferior electrode on the right gluteal member longitudinally inferior of the right intermediate electrode; and
a connective element on the recipient side of the dressing.

**49.** The dressing of clause **48** comprising a sacral member laterally between the left and right gluteal members.
**50.** The dressing of clause **48** including an exciter capable of selectively applying an intermittent excitation in each excitation mode set forth below:

| Mode | Involved Electrodes | Relative Polarity |
|---|---|---|
| A | none | not applicable |
| B | left intermediate / left inferior | either |
| C | right intermediate / right inferior | either |
| D1 | left intermediate / left inferior and right intermediate / right inferior | same left and right |
| D2 | left intermediate / left inferior and right intermediate / right inferior | opposite left and right |
| E | left intermediate / right intermediate | either |

**51.** The dressing of clause **50** wherein the dressing is sized and the electrodes are positioned such that when the dressing is applied to a care recipient having a target anatomy with each gluteal member approximately laterally centered on a corresponding gluteus maximus of the care recipient, the excitation stimulates one or more muscles of the care recipient as set forth below:

| Mode | Involved Electrodes | Relative Polarity | Primary Muscle Stimulated | Secondary Muscle Stimulated |
|---|---|---|---|---|
| A | none | not applicable | none | none |
| B | left intermediate / left inferior | either | Left gluteus maximus | |
| C | right intermediate / right inferior | either | Right gluteus maximus | |
| D1 | left intermediate / left inferior and right intermediate / right inferior | same left and right | Left gluteus maximus and right gluteus maximus | |
| D2 | left intermediate / left inferior and right intermediate / right inferior | opposite left and right | Left gluteus maximus and right gluteus maximus. | superior region of left and right gluteus medius (weak) |
| E | left intermediate / right intermediate | either | superior region of left and right gluteus medius (not as weak as mode D2 for a given voltage) | |

**52.** The dressing of clause **49** wherein the left and right gluteal members each have an inferior terminus, the left and right inferior electrodes are positioned near the termini of the left and right gluteal members respectively, and the left and right intermediate electrodes are positioned in approximate longitudinal alignment with the sacral member.
**53.** The dressing of clause **48** including a left superior electrode longitudinally superior of the left intermediate electrode and a right superior electrode longitudinally superior of the right intermediate electrode.
**54.** The dressing of clause **53** including an exciter capable of selectively applying an intermittent excitation in each excitation mode set forth below:

| Mode | Involved Electrodes | Relative Polarity |
|---|---|---|
| A | none | either |
| B | left intermediate / left inferior | either |
| C | right intermediate / right inferior | either |
| D1 | left intermediate / left inferior and right intermediate / right inferior | same left and right |
| D2 | left intermediate / left inferior and right intermediate / right inferior | opposite left and right |
| E | left superior / right superior | either |

**55.** The dressing of clause **54** wherein the dressing is sized such that when the dressing is applied to a care recipient having a target anatomy with each gluteal member approximately laterally centered on a corresponding gluteus maximus of the care recipient, the excitation stimulates one or more muscles of the care recipient as set forth below.

| Mode | Involved Electrodes | Relative Polarity | Primary Muscle Stimulated | Secondary Muscle Stimulated |
|---|---|---|---|---|
| A | none | not applicable | none | none |
| B | left intermediate / left inferior | either | Left gluteus maximus | |
| C | right intermediate / right inferior | either | Right gluteus maximus | |
| D1 | left intermediate / left inferior and right intermediate / right inferior | same left and right | Left gluteus maximus and right gluteus maximus | |
| D2 | left intermediate / left inferior and right intermediate/ right inferior | opposite left and right | Left gluteus maximus and right gluteus maximus | superior region of left and right gluteus medius (weak) |
| E | left superior / right superior | either | superior region of left and right gluteus medius | |

**56.** The dressing of clause **49** wherein the left and right gluteal members each have an inferior terminus, the left and right inferior electrodes are positioned near the termini of the left and right gluteal members respectively, and the left and right superior electrodes are positioned in approximate longitudinal alignment with the sacral member.

## Claims

1. A preventive dressing (100) having a recipient side (112), an environmental side (114), and a perimeter (P), the dressing (100) comprising:
A) a moisture barrier layer having(110):
a) a sacral member (130) having a longitudinal length (L_{SM}) and an inferior edge (132);
b) a left gluteal member (136) having a base (138) which borders a left side (140) of the sacral member (130), and a left extension (142) which extends longitudinally inferiorly further than the inferior edge (132) by a left gluteal extension dimension (L_{EXT}) which is approximately no less than the longitudinal length (L_{SM}) of the sacral member (130);
c) a right gluteal member (156) having a base (158) which borders a right side (160) of the sacral member (130), and a right extension (162) which extends longitudinally inferiorly further than the inferior edge (132) by a right gluteal extension dimension (L_{EXT}) which is approximately no less than the longitudinal length (L_{SM}) of the sacral member (130) so that the left gluteal member and the right gluteal member define an intermember space (180); and
B) a connective element (120) on the recipient side of the dressing (112), the connective element (120) being arranged to provide a zone of occlusion (Z).

2. The dressing of claim 1 wherein the connective element comprises an adhesive.

3. The dressing of either claim 1 or claim 2 wherein the connective element is a continuous strip of adhesive extending along substantially the perimeter of the dressing.

4. The dressing of claim 3 wherein the adhesive strip borders the occlusion zone and wherein an auxiliary layer resides in the occlusion zone, and the auxiliary layer comprises at least one of:
a liquid absorbant layer,
a liquid permeable layer,
a pressure equalizing layer.

5. The dressing of either claim 1 or claim 2 wherein the connective element comprises a sacral adhesive layer and a gluteal adhesive element and wherein:
the sacral adhesive layer is an adhesive which forms substantially all of:
a) the recipient side of the sacral member, and
b) the recipient sides of the left and right gluteal bases,
the gluteal extensions have a margin and the gluteal adhesive element is a continuous strip of adhesive extending along substantially the margin of the gluteal member extensions.

6. The dressing of either claim 1 or claim 2 wherein the connective element is an adhesive which forms substantially all of the recipient side of the dressing.

7. The dressing of any preceding claim including at least one auxiliary layer residing in the zone of occlusion.

8. The dressing of any preceding claim including a pressure equalizing layer spanning laterally across a longitudinally extending centerline of the dressing in the zone of occlusion to define a pressure equalizing zone, the pressure equalizing layer imparting a stiffness to the pressure equalizing zone which is greater than the stiffness of the dressing laterally to the left and right of the pressure equalizing zone.

9. The dressing of any preceding claim including a pressure equalizing zone spanning laterally across a longitudinally extending centerline of the dressing in the zone of occlusion, the pressure equalizing zone having a planform and a thickness which is greater than the thickness of the dressing outside the planform of the pressure equalizing zone.

10. The dressing of any preceding claim including a zone spanning laterally across a longitudinally extending centerline of the dressing in the zone of occlusion, the zone having a customizable thickness.

11. The dressing of any preceding claim including a classified zone spanning laterally across a longitudinally extending centerline of the dressing in the zone of occlusion, the classified zone having a class specific thickness which is one of a set of two or more class specific thicknesses.

12. The dressing of any preceding claim including a moisture management layer in the zone of occlusion.

13. The dressing of any one of claims 1 to 11 including a moisture management layer and a pressure equalizing layer both of which are in the zone of occlusion and transversely between the moisture barrier layer and the connective element.

14. The dressing of any preceding claim comprising a tab which extends inferiorly from the sacral member at a location between the gluteal members, the tab having a longitudinal dimension which is less than the longitudinal dimension of the extensions.

15. The dressing of any preceding claim comprising a tab which extends inferiorly from the sacral member at a location between the gluteal members, the tab being sized and shaped to conform to the intergluteal cleft of a target care recipient thereby preserving occlusivity of the zone of occlusion.

16. The dressing of any preceding claim wherein dimensions of the dressing are as set forth in the table below in which
W_{S,IT} is a lateral dimension of the intermember space taken at a longitudinal position corresponding to the longitudinal position of the ITs of the anatomy of a target care recipient when the dressing is applied to the anatomy with its superior edge approximately longitudinally aligned with the sacral base of the anatomy and
L_{GM} is the longitudinal dimension of the left and right gluteal members:
| dimension | value |
|---|---|
| W_{S,IT} | less than distance between left and right ischial tuberosity of the target anatomy. |
| L_{GM} | greater than or equal to a longitudinal distance L_{L5S1-IT} from the ischial tuberosity to the L5/S1 interface of the target anatomy. |

17. The dressing of any preceding claim wherein at least part of the sacral member has a shape which approximates the shape of a triangle having a base and an apex, the base being superior of the apex.

18. The dressing of any preceding claim wherein the sacral member, the left gluteal member and the right gluteal member are individual members and wherein the left and right gluteal members are dimensioned such that the extensions thereof extend inferiorly of the inferior edge of the sacral member when the gluteal members are applied to a target care recipient such that the gluteal members border the sacral member and the sacral member is longitudinally aligned with the care recipient's sacrum.

## Patentansprüche

1. Ein vorbeugender Verband (100) mit einer Empfängerseite (112), einer Umgebungsseite (114) und einer Umgrenzungslinie (P), wobei der Verband (100) umfasst:
A) eine Feuchtigkeitsbarriereschicht (110) mit:
a) einem Element für den Kreuzbeinbereich (130) mit einer längsgerichteten Länge (L_{SM}) und einem unteren Rand (132);
b) einem Element für den linken Gesäßbereich (136) mit einer Basis (138), die an eine linke Seite (140) des Elements für den Kreuzbeinbereich (130) angrenzt, und einer linken Verlängerung (142), die sich in Längsrichtung unterhalb weiter als der untere Rand (132) um eine Abmessung der Verlängerung für den linken Gesäßbereich (L_{EXT}) erstreckt, die ungefähr nicht weniger als die längsgerichtete Länge (L_{SM}) des Elements für den Kreuzbeinbereich (130) beträgt;
c) einem Element für den rechten Gesäßbereich (156) mit einer Basis (158), die an eine rechte Seite (160) des Elements für den Kreuzbeinbereich (130) angrenzt, und einer rechten Verlängerung (162), die sich in Längsrichtung unterhalb weiter als der untere Rand (132) um eine Abmessung der Verlängerung für den rechten Gesäßbereich (L_{EXT}) erstreckt, die ungefähr nicht weniger als die längsgerichtete Länge (L_{SM}) des Elements für den Kreuzbeinbereich (130) beträgt, sodass das Element für den linken Gesäßbereich und das Element für den rechten Gesäßbereich einen Elementzwischenraum (180) definieren; und
B) ein verbindendes Element (120) auf der Empfängerseite des Verbandes (112), wobei das verbindende Element (120) so angeordnet ist, dass eine Okklusionszone (Z) bereitgestellt wird.

2. Der Verband nach Anspruch 1, wobei das verbindende Element einen Klebstoff umfasst.

3. Der Verband nach entweder Anspruch 1 oder Anspruch 2, wobei das verbindende Element ein durchgehender Klebstoffstreifen ist, der sich im Wesentlichen entlang der Umgrenzungslinie des Verbandes erstreckt.

4. Der Verband nach Anspruch 3, wobei der Klebstoffstreifen an die Okklusionszone angrenzt und wobei sich eine zusätzliche Schicht in der Okklusionszone befindet, und die zusätzliche Schicht mindestens umfasst:
eine flüssigkeitsabsorbierende Schicht,
eine flüssigkeitsdurchlässige Schicht,
eine druckausgleichende Schicht.

5. Der Verband nach entweder Anspruch 1 oder Anspruch 2, wobei das verbindende Element eine Klebstoffschicht für den Kreuzbeinbereich und ein Klebstoffelement für den Gesäßbereich umfasst und wobei:
die Klebstoffschicht für den Kreuzbeinbereich ein Klebstoff ist, der im Wesentlichen alles bildet von:
a) der Empfängerseite des Elements für den Kreuzbeinbereich, und
b) den Empfängerseiten der linken und rechten Gesäßbasisbereiche,
die Verlängerungen der Gesäßbereiche einen Rand aufweisen und das Klebstoffelement für den Gesäßbereich ein durchgehender Klebstoffstreifen ist, der sich im Wesentlichen entlang des Randes der Verlängerungen des Gesäßbereichs erstreckt.

6. Der Verband nach entweder Anspruch 1 oder Anspruch 2, wobei das verbindende Element ein Klebstoff ist, der im Wesentlichen alles von der Empfängerseite des Verbandes bildet.

7. Der Verband nach einem der vorstehenden Ansprüche, der mindestens eine zusätzliche Schicht enthält, die sich in der Okklusionszone befindet.

8. Der Verband nach einem der vorstehenden Ansprüche, der eine druckausgleichende Schicht enthält, die sich seitlich über eine in Längsrichtung verlaufende Mittellinie des Verbandes in die Okklusionszone erstreckt, um eine druckausgleichende Zone zu definieren, wobei die druckausgleichende Schicht der druckausgleichenden Zone eine Steifheit verleiht, die größer ist als die Steifheit des Verbandes links und rechts seitlich von der druckausgleichenden Zone.

9. Der Verband nach einem der vorstehenden Ansprüche, der eine druckausgleichende Zone enthält, die sich seitlich über eine in Längsrichtung verlaufende Mittellinie des Verbandes in die Okklusionszone erstreckt, wobei die druckausgleichende Zone eine Grundform und eine Stärke aufweist, die größer ist als die Stärke des Verbandes außerhalb der Grundform der druckausgleichenden Zone.

10. Der Verband nach einem der vorstehenden Ansprüche, der eine Zone enthält, die sich seitlich über eine in Längsrichtung verlaufende Mittellinie des Verbandes in die Okklusionszone erstreckt, wobei die Zone eine anpassbare Stärke aufweist.

11. Der Verband nach einem der vorstehenden Ansprüche, der eine klassifizierte Zone enthält, die sich seitlich über eine in Längsrichtung verlaufende Mittellinie des Verbandes in die Okklusionszone erstreckt, wobei die klassifizierte Zone eine klassenspezifische Stärke aufweist, die eine aus einem Satz von zwei oder mehr klassenspezifischen Stärken ist.

12. Der Verband nach einem der vorstehenden Ansprüche, der eine Schicht zum Feuchtigkeitsmanagement in der Okklusionszone enthält.

13. Der Verband nach einem der Ansprüche 1 bis 11, der eine Schicht zum Feuchtigkeitsmanagement und eine druckausgleichende Schicht enthält, die sich beide in der Okklusionszone und quer zwischen der Feuchtigkeitsbarriereschicht und dem verbindenden Element befinden.

14. Der Verband nach einem der vorstehenden Ansprüche, der eine Lasche umfasst, die sich unterhalb von dem Element für den Kreuzbeinbereich an einer Stelle zwischen den Elementen für den Gesäßbereich erstreckt, wobei die Lasche eine Abmessung in Längsrichtung aufweist, die geringer ist als die längsgerichtete Abmessung der Verlängerungen.

15. Der Verband nach einem der vorstehenden Ansprüche, der eine Lasche umfasst, die sich unterhalb von dem Element für den Kreuzbeinbereich an einer Stelle zwischen den Elementen für den Gesäßbereich erstreckt, wobei Größe und Form der Lasche so ausgelegt sind, dass sie der Gesäßfalte eines anvisierten Pflegebedürftigen entspricht und dadurch die Okklusivität der Okklusionszone aufrechterhalten wird.

16. Der Verband nach einem der vorstehenden Ansprüche, wobei die Abmessungen des Verbandes wie in der unten aufgeführten Tabelle sind, in der
W_{S,1T} eine seitliche Abmessung eines Elementzwischenraums ist, die an einer Position in Längsrichtung abgenommen wird, die der längsgerichteten Position der Sitzbeinhöcker der Anatomie eines anvisierten Pflegebedürftigen entspricht, wenn der Verband auf der Anatomie mit seinem oberen Rand etwa längs an der Kreuzbeinbasis der Anatomie ausgerichtet ist, und
LGM die längsgerichtete Abmessung der Elemente für den linken und rechten Gesäßbereich ist:
| Abmessung | Wert |
|---|---|
| W_{S,IT} | geringer als der Abstand zwischen dem linken und rechten Sitzbeinhöcker der Zielanatomie. |
| L_{GM} | größer als ein oder gleich einem Abstand in Längsrichtung L_{L5S1-IT} vom Sitzbeinhöcker zum L5/S1-Übergang der Zielanatomie. |

17. Der Verband nach einem der vorstehenden Ansprüche, wobei mindestens ein Teil des Elements für den Kreuzbeinbereich eine Form aufweist, die ungefähr der Form eines Dreiecks mit einer Basis und einer Spitze entspricht, wobei die Basis oberhalb der Spitze liegt.

18. Der Verband nach einem der vorstehenden Ansprüche, wobei das Element für den Kreuzbeinbereich, das linke Element für den Gesäßbereich und das rechte Element für den Gesäßbereich einzelne Elemente sind und wobei die Elemente für den linken und rechten Gesäßbereich so bemessen sind, dass die Verlängerungen davon unterhalb des unteren Randes des Elements für den Kreuzbeinbereich verlaufen, wenn die Elemente für den Gesäßbereich bei einem anvisierten Pflegebedürftigen so angebracht werden, dass die Elemente für den Gesäßbereich an das Element für den Kreuzbeinbereich angrenzen und das Element für den Kreuzbeinbereich in Längsrichtung am Kreuzbein des Pflegebedürftigen ausgerichtet ist.

## Revendications

1. Un pansement préventif (100) possédant une face bénéficiaire (112), une face environnement (114), et un périmètre (P), le pansement (100) comprenant :
A) une couche anti-humidité possédant (110) :
a) un élément sacral (130) possédant une longueur longitudinale (L_{ES}) et un bord inférieur (132) ;
b) un élément fessier gauche (136) possédant une base (138) qui borde le côté gauche (140) de l'élément sacral (130), et une extension gauche (142) qui prolonge longitudinalement la partie inférieure au-delà du bord inférieur (132) par une dimension d'extension du fessier gauche (L_{EXT}) qui n'est approximativement pas inférieure à la longueur longitudinale (L_{ES}) de l'élément sacral (130) ;
c) un élément fessier droit (156) possédant une base (158) qui borde le côté droit (160) de l'élément sacral (130), et une extension droite (162) qui prolonge longitudinalement la partie inférieure au-delà du bord inférieur (132) par une dimension d'extension du fessier droit (L_{EXT}) qui n'est approximativement pas inférieure à la longueur longitudinale (L_{ES}) de l'élément sacral (130) de manière à ce que l'élément fessier gauche et l'élément fessier droit définissent un espace interélément (180) ; et
B) un élément de raccordement (120) sur la face bénéficiaire du pansement (112), l'élément de raccordement (120) étant prévu pour fournir une zone d'occlusion (Z).

2. Le pansement selon la revendication 1 dans lequel l'élément de raccordement comprend un adhésif.

3. Le pansement selon la revendication 1 ou la revendication 2 dans lequel l'élément de raccordement est une bande continue d'adhésif s'étendant substantiellement le long du périmètre du pansement.

4. Le pansement selon la revendication 3 dans lequel la bande adhésive borde la zone d'occlusion et dans lequel une couche supplémentaire est située dans la zone d'occlusion, et la couche supplémentaire comprend au moins l'un des éléments suivants :
une couche d'absorption de liquide,
une couche perméable au liquide,
une couche d'équilibrage de la pression.

5. Le pansement selon la revendication 1 ou la revendication 2 dans lequel l'élément de raccordement comprend une couche adhésive sacrale et un élément adhésif de fessier et dans lequel :
la couche adhésive sacrale est un adhésif qui forme substantiellement l'ensemble de :
a) la face bénéficiaire de l'élément sacral, et
b) les faces bénéficiaire des bases fessier gauche et droite,
les extensions fessier ont une bordure et l'élément adhésif du fessier est une bande continue d'adhésif s'étendant substantiellement le long de la bordure des extensions de l'élément fessier.

6. Le pansement selon la revendication 1 ou la revendication 2 dans lequel l'élément de raccordement est un adhésif qui forme substantiellement l'ensemble de la face bénéficiaire du pansement.

7. Le pansement selon une quelconque revendication précédente comprenant au moins une couche supplémentaire se situant dans la zone d'occlusion.

8. Le pansement selon une quelconque revendication précédente comprenant une couche d'équilibrage de la pression couvrant de part en part une ligne centrale longitudinale du pansement dans la zone d'occlusion afin de définir une zone d'équilibrage de la pression, la couche d'équilibrage de la pression conférant une rigidité à la zone d'équilibrage de la pression qui est supérieure à la rigidité du pansement sur les côtés gauche et droit de la zone d'équilibrage de la pression.

9. Le pansement selon une quelconque revendication précédente comprenant une zone d'équilibrage de la pression couvrant de part en part une ligne centrale longitudinale du pansement dans la zone d'occlusion, la zone d'équilibrage de la pression possédant une forme et une épaisseur supérieure à l'épaisseur du pansement à l'extérieur de la forme de la zone d'équilibrage de la pression.

10. Le pansement selon une quelconque revendication précédente comprenant une zone couvrant de part en part une ligne centrale longitudinale du pansement dans la zone d'occlusion, la zone ayant une épaisseur adaptable.

11. Le pansement selon une quelconque revendication précédente comprenant une zone classifiée couvrant de part en part une ligne centrale longitudinale du pansement dans la zone d'occlusion, la zone classifiée possédant une épaisseur spécifique classifiée qui est l'une de deux, ou plus, épaisseurs spécifiques classifiées.

12. Le pansement selon une quelconque revendication précédente comprenant une couche de gestion d'humidité dans la zone d'occlusion.

13. Le pansement selon l'une quelconque des revendications 1 à 11 comprenant une couche de gestion d'humidité et une couche d'équilibrage de la pression, les deux étant situées dans la zone d'occlusion et transversalement entre la couche anti-humidité et l'élément de raccordement.

14. Le pansement selon une quelconque revendication précédente comprenant une languette qui s'étend en dessous de l'élément sacral dans un endroit situé entre les éléments fessiers, la languette ayant une dimension longitudinale inférieure à la dimension longitudinale des extensions.

15. Le pansement selon une quelconque revendication précédente comprenant une languette qui s'étend en dessous de l'élément sacral dans un endroit situé entre les éléments fessiers, la languette étant dimensionnée et sa forme conçue pour s'adapter au sillon interfessier du bénéficiaire de soins cible, préservant ainsi le caractère occlusif de la zone d'occlusion.

16. Le pansement selon une quelconque revendication précédente dans lequel les dimensions du pansement sont indiquées dans le tableau ci-dessous dans lequel
I_{S,TI} est la dimension latérale de l'espace interélément mesurée en position longitudinale correspondant à la position longitudinale des TI de l'anatomie du bénéficiaire de soins cible lorsque le pansement est appliqué à l'anatomie avec son bord supérieur approximativement aligné longitudinalement avec la base sacrale de l'anatomie et
L_{EF} est la dimension longitudinale des éléments fessiers droit et gauche :
| dimension | valeur |
|---|---|
| I_{S,TI} | inférieure à la distance de la tubérosité ischiatique droite et gauche de l'anatomie cible. |
| L_{EF} | supérieure ou égale à la distance longitudinale LL_{5S1-TI} de la tubérosité ischiatique à l'interface L5/S1 de l'anatomie cible. |

17. Le pansement selon une quelconque revendication précédente dans lequel au moins une partie de l'élément sacral a une forme qui s'approche de la forme d'un triangle ayant une base et une pointe, la base étant supérieure à la pointe.

18. Le pansement selon une quelconque revendication précédente dans lequel l'élément sacral, l'élément fessier gauche et l'élément fessier droit sont des éléments individuels et dans lequel les éléments fessiers droit et gauche sont dimensionnés de manière à ce que les extensions de ceux-ci s'étendent en dessous du bord inférieur de l'élément sacral lorsque les éléments fessiers sont appliqués sur un bénéficiaire de soins cible de manière à ce que les éléments fessiers bordent l'élément sacral et que l'élément sacral soit aligné longitudinalement avec le sacrum du bénéficiaire de soins
